# EUROPEAN PATENT APPLICATION

(11) **EP 1 314 740 A1**
(43) Date of publication of application: **28.05.2003**
(21) Application number: 01936974.3
(22) Date of filing: 12.06.2001
(51) Int. Cl.: C07K 16/00, C12N 15/09

(54) **METHOD OF CONSTRUCTING scFv ANTIBODY FUSED WITH FLUORESCENT PROTEIN**

(30) Priority: 14.06.2000 JP 2000178880
(71) Applicant: Medical & Biological Laboratories Co., Ltd., Nagayo-shi, Aichi 460-0002 (JP)
(72) Inventor: MORINO, Kazuhiko, Toyoake-shi, Aichi 470-1123 (JP); AKAHORI, Yasushi, Nagoya-shi, Aichi 468-0056 (JP); IBA, Yoshitaka, Nagoya-shi, Aichi 458-0002 (JP); SHINOHARA, Midori, Toyoake-shi, Aichi 470-1131 (JP); UKAI, Yoshinori, Ekoserunimuradai 1-F, Toyoake-shi, Aichi 470-1101 (JP); KUROSAWA, Yoshikazu, Nagoya-shi, Aichi 465-0075 (JP)
(74) Representative: Bösl, Raphael, Dr. rer. nat., Dipl.-Chem.
(86) International application number: JP0104964
(87) International publication number: WO01096401

(57) **Abstract**

A method for preparing a scFv antibody with which a fluorescent protein has been fused and which has an antigen binding activity is provided. A scFv antibody library composed of phage clones expressing a scFv antibody on the surface is prepared, and from this, a clone expressing an antibody for recognizing the specific antigen is selected. Then, a scFv antibody gene is acquired from the relevant clone and this is introduced into an expression vector. A gene coding for a fluorescent protein has been previously incorporated in the expression vector, a scFv antibody with which a fluorescent protein has been fused is produced by expressing the vector in a host.

## Description

### FIELD OF THE INVENTION

The present invention relates to a method for preparing an antibody capable of emitting the fluorescence. More particularly, the present invention relates to a method for preparing an scFv antibody with which a fluorescent protein has been fused.

### BACKGROUND ART

As a method for preparing a library of antibodies, the phage display method has been utilized. The phage display method has been contrived by Smith in 1985 (Smith, G.P., Science 228:4075, 1315-7, 1985), a filamentous bacteriophage having a single strand DNA such as M13 phage is used. A phage particle consisting of a protein referred to as cp8 which surrounds DNA and constitutes the most part of a phage particle and a protein referred to as cp3, 5 pieces of the cp3 which function when the phage is infected with *E. coli,* and the like. A system which constructs a gene so as to code a polypeptide in a form for fusing with this cp3 or cp8 and expresses the protein on the surface of a phage particle is referred to as the phage display system. A phage particle holding a protein having the binding activity with the other substances on the surface can be condensed by utilizing the binding activity with its ligand. Thus, a method for condensing a phage particle having the objective binding activity is referred to as the panning method. In a condensed phage particle, DNA coding for a protein having a necessary binding activity is packaged. Thus, a system capable of efficiently carrying out a screening based on the binding activity and cloning of DNA by utilizing a filamentous phage has been realized (McCafferty J, Griffiths, A.D, Winter, G, Chiswell, D. J., Nature, 348: 630, 1552-4, 1990).

The phage display system has been applied to an antibody, only VH domain, scFv, Fv, and Fab type antibodies have been expressed in a form that cp3 or cp8 is fused with. A phage antibody binding to an antigen contains a gene coding for the antibody at the same time. Specifically, it is a tremendous advantage in the point that in the phage display system, an antibody molecule capable of binding to the specific antigen and a gene coding for the antibody molecule are acquired at the same time. However, there have been many antibodies isolated from the antibody libraries prepared at an early stage, which were using the phage display system, whose antigen binding avidity was low. As an attempt for enhancing the binding avidity, a method for artificially giving the mutation to a gene was proposed. Winter, et al. has obtained an antibody library capable of acquiring the antibody excellent in the affinity by preparing an antibody library having a semi-artificial sequence into which a randomized sequence has been inserted between whole of the isolated VH, VL genes and JH, JL genes (Nissim A, Winter G etal. EMBO J. 13:3 692-8, 1994). De Kruif et al. also have prepared an antibody library on the basis of fundamentally the same principle (de Kruif J, Boel E, Logtenberg, T., J.Mol. Biol., 248:1 97-105, 1995). Vaughan et al. attempted to secure a sufficiently large antibody repertory by expanding the size of the library in 1996 (Vaughan, T. J. et al. Nat. Biotechnol. 14: 3 309-14, 1996). Moreover, the present inventors have proposed a method for providing an antibody library having more various repertories and containing an antibody molecule maintaining its functional conformation at a high ratio (see Japanese Patent Application No.H12-050543).

As described above, according to a phage display system makes it possible to acquire not only an antibody molecule but also a gene coding for the antibody molecule at the same time. Since both ends of this gene can be designed as a unique res triction enzyme site, by means of a simple operation such that a phage DNA is isolated, the restriction enzyme treatment is performed, and subsequently, it is incorporated into a suitable expression vector, the expression form of the relevant gene can be converted from the phage display type into another form (Ito, W. and Kurosawa, Y.: J. Biol. Chem., 268:20668-20675, 1993).

On the other hand, a protein referred to as GFP (green fluorescent protein), which is capable of emitting fluorescence, has been known, and the labeling of a variety of proteins with a GFP has been attempted. The GFP is a protein molecule capable of emitting fluorescence by irradiating a light having the specific wavelength, a special reagent or the like is not required to detect the protein molecule labeled with the GFP. Therefore, needless to say that it can be rapidly and easily detected, and it has also an advantage that the detection can be directly carried out even in the living cell or the like, further applications are expected. It should be noted that as a protein emitting fluorescence similarly to GFP, RFP (red fluorescent protein), BFP (blue fluorescent protein), YFP (yellow fluorescent protein), CFP (cyan fluorescent protein) and mutants thereof have been developed (Crameri, A. et al.: Nature Biotechnol., 14: 315-319, 1996; Yang, T. et al. : J. Biol. Chem., 273: 8212-8216, 1998; Lybarger, L. et al.: Cytometry, 31: 147-152, 1998).

Concerning antibody molecules, the labeling with GFP or the like has been also considered, however, it has been pointed out that the following problems exist.

As for S-S binding in antibody molecule(s), there are two kinds of S-S bindings, a S-S binding in a molecule within a domain and a S-S binding between molecules, that is, a S-S binding between a H-chain and a L-chain or a S-S binding between a H-chain and a H-chain. Antibodies expressed in the cytoplasm of bacteria have not indicated the antigen binding activity so much at the time when the recombinant genetic technology has been initially applied to the production of an antibody. It is because under the conditions within the cytoplasm, the correct folding of polypeptide does not occur, and further, a S-S binding is not correctly formed (Boss, M. A., Kenten, J.H. , Wood, C. R., Emtage, J. S., Nucleic Acids Res., 12: 3791-806, 1984). In contrast to this, the attempt that pelB sequence and ompA sequence are introduced into N-terminal side of a polypeptide so that the translated polypepride passes though the membrane and secreted into the periplasm has been performed, however, either of Fab fragment or scFv fragment whose binding with its antigen is appropriately performed could not be obtained (Skerra, A., and Pluckthun, A.: Science 240:1038-1041, 1988; Better, M., Chang, C.P. , Robinson, R. R., andHorwitz, A. R.: Science 240: 1041-1043, 1988). In order to create an antibody which emits fluorescence by fusing with GFP, both of the conflicting conditions, one of which is necessary for antibody to obtain the antigen binding activity and the other is necessary for GFP to emit fluorescence must be fulfilled. GFP cannot emit the fluorescence under the conditions immediately after it has been translated within bacteria. Although other factors are not required, the luminescence center is formed by multistage chemical reactions occurring with in the molecule, thereby resulting in being capable of emitting the fluorescence (Reid, B., and Flynn, G. C.: Biochemistry 36: 6786-6791, 1997).

On the other hand, recently, owing to the development of the intra-body technology, a scFv antibody having an antigen binding ability has been capable of being well expressed within cytoplasm in an animal cell.

### SUMMARY OF THE INVENTION

In such a situation, the present inventors have directed their attention to scFv antibodies, and attempted to label a scFv antibody with fluorescent protein molecules. As a result, the present inventors have succeeded in obtaining a scFv antibody labeled with a fluorescent protein by utilizing a system using in combination with the phage display system and have completed the present invention. The constitution of the present inventions are as follows:
[1] A method for preparing a scFv antibody with which a fluorescent protein has been fused, comprising the following steps:
   1) a step of preparing a scFv antibody library composed of phage clones, each one of which expresses a scFv antibody on the surface;
   2) a step of selecting a phage clone expressing a scFv antibody capable of binding to the relevant antigen by screening the scFv antibody library with an antigen;
   3) a step of acquiring the gene coding for the scFv antibody from the phage clone which has been selected in the step 2);
   4) a step of incorporating the relevant gene into an expression vector capable of expressing a fusion protein of the expression product of the relevant gene and a fluorescent protein by incorporating the gene acquired in step 3); and
   5) a step of transforming a host using the recombinant vector obtained in step 4) and expressing the fusion protein.
[2] The method for preparing a scFv antibody set forth in [1] wherein the foregoing scFv antibody library contains at least one portion of light chain variable region selected so as to reconstruct a functional conformation with a heavy variable region.
[3] The method for preparing a scFv antibody set forth in [1] or [2], wherein the scFv antibody on the surface of each phage clone in the scFv antibody library has a VH region, a VL region, a linker and a CL region.
[4] The method for preparing a scFv antibody set forth in any one of [1] to [3], wherein the foregoing fluorescent protein is one or two or more of fluorescent protein(s) selected from the group consisting of GFP, RFP, BFP, YFP, CFP and mutants thereof.
[5] A scFv antibody with which a fluorescent protein has been fused, prepared by the method set forth in any one of [1] to [4].
[6] A scFv antibody with which a fluorescent protein has been fused, wherein the scFv antibody has a Fv region which consists of a VH region, VL region, and a linker, a CL region, and a fluorescent protein connected to the Fv region via the CL region.
[7] The scFv antibody set forth in [6], wherein the foregoing fluorescent protein is one or two or more of fluorescent protein(s) selected from the group consisting of GFP, RFP, BFP, YFP, CFP and mutants thereof.
[8] An immunological measurement method using the scFv antibody set forth in any one of [5] to [7].
[9] A recombinant vector having a base sequence coding for a scFv antibody gene and the base sequence coding for a fluorescent protein, which are acquired by the steps of 1) to 4) set forth in [1].
[10] The recombinant vector set forth in [9], wherein the scFv antibody gene consists of a VH gene, a linker sequence, a VL gene and a CL gene.
[11] The recombinant vector set forth in any one of [9] or [10], wherein the fluorescent protein is one or two or more of fluorescent protein(s) selected from the group consisting of GFP, RFP, BFP, YFP, CFP and mutants thereof.
[12] A recombinant vector having the following structure: 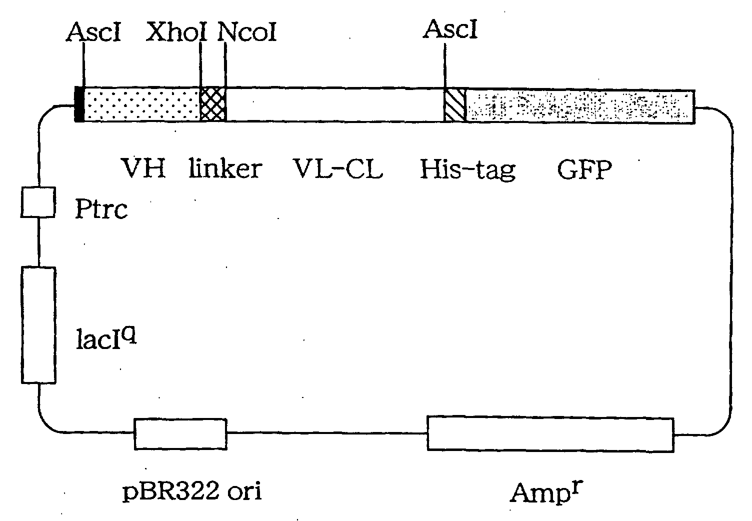
[13] An expression vector having a start codon, a site to which a scFv antibody gene is introduced, and base sequence coding for a fluorescent protein in order starting from 5' end side.
[14] The expression vector set forth in [13], wherein the scFv antibody gene consists of a VH gene, a linker sequence, a VL gene and a CL gene.
[15] An expression vector set forth in [13] or [14] wherein the foregoing expression vector has a base sequence coding for His-tag, myc-tag or HA-tag between the site to which the foregoing scFv antibody gene is introduced and the base sequence coding for the foregoing fluorescent protein.
[16] The expression vector set forth in any one of [13] through [15], wherein the foregoing fluorescent protein is one or two or more of fluorescent protein(s) selected from the group consisting of GFP, RFP, BFP, YFP, CFP and mutants thereof.
[17] A kit for preparing a scFv antibody with which a fluorescent protein has been fused, comprising:
   a scFv antibody library composed of phage clones, each one of which expresses a scFv antibody on the surface; and
   an expression vector capable of expressing a fusion protein of the expression product of the relevant gene and a fluorescent protein by incorporating the scFv antibody gene.
[18] The kit set forth in [17], wherein the scFv antibody gene consists of a VH region, VL region, a linker sequence, a CL region.
[19] A kit for preparing a scFv antibody with which a fluorescent protein is fused, containing a scFv antibody gene library composed of phage clones having a scFv antibody gene or phagemid clones having a scFv antibody gene, and a vector capable of expressing a fusion protein of the expression product of the relevant gene and a fluorescent protein by incorporating a scFv antibody gene.
[20] The kit set forth in [19], wherein the scFv antibody gene consists of a VH gene, a VL gene, a linker sequence and a CL gene.
[21] The kit set forth in any one of [17] to [20], wherein the vector is an expression vector set forth in any one of [13] to [16].

### BRIEF DESCRIPTION OF THE DRAWINGS

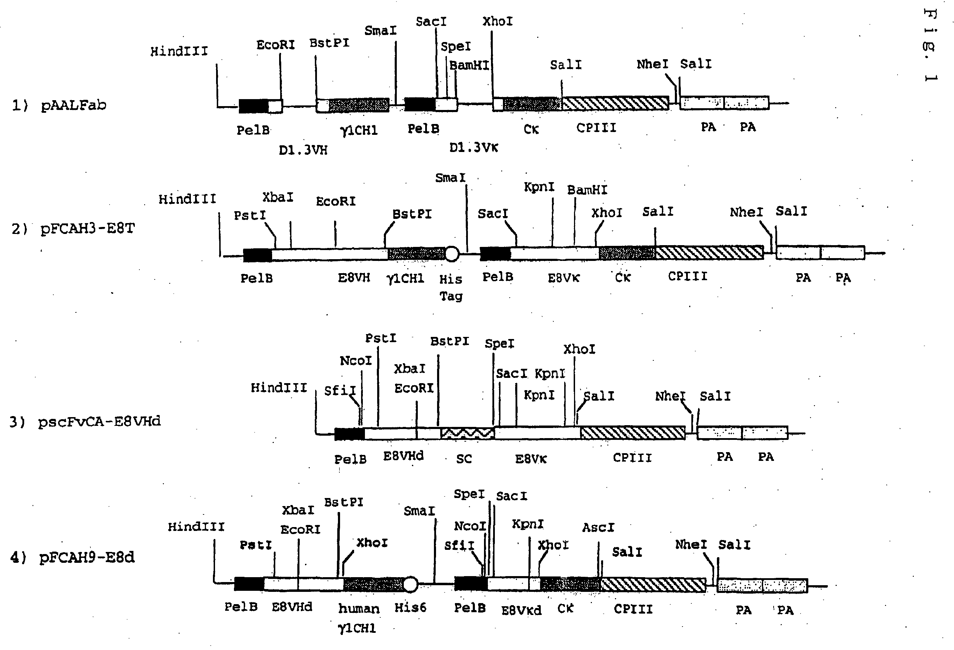

Fig. 1 is a diagram schematically showing the structures of a variety of vectors used for the preparation of a variable region library in Example of the present invention;
   1) pAALFab: vector for D1.3 mutation
   2) pFCAH3-E8T: vector for E8 expression. Restriction enzyme site was altered based on pAALFab. PstI, XbaI and KpnI sites were newly added and the locations of EcoRI and XhoI sites were changed.
   3) PFvCA-E8VHd: vector for heavy chain variable region gene cloning. Restriction enzyme sites were altered based on pFCAH3-E8T. The portion between XbaI-EcoRI was deleted, and KpnI, SfiI, NcoI and SpeI sites were newly added. A heavy chain variable region gene is capable of being cloned into SfiI-XhoI site.
   4) pFCAH9-E8d: vector for heavy chain variable region gene cloning. DNA sequence was altered based on pFCAH3 -E8T and pFvCA-E8VHd. MouseγCH1 was replaced with human γCH1. SfiI, NcoI and AscI sites were newly added. A light chain variable region is capable of being cloned into SfiI-AscI site.
Fig. 2 is a diagram showing the base sequence of an insert of pFCAH9-E8d.
Fig. 3 is a diagram showing the restriction enzyme site of an insert of pFCAH9-E8d and the amino acid sequence coded by the base sequence (1).
Fig. 4 is a diagram showing the restriction enzyme site of an insert of pFCAH9-E8d and the amino acid sequence coded by the base sequence (2).
Fig. 5 is a diagram showing the restriction enzyme site of an insert of pFCAH9-E8d and the amino acid sequence coded by the base sequence (3).
Fig. 6 is a diagram showing the base sequence of an insert of pscFvCA-E8VHd.
Fig. 7 is a diagram showing the restriction enzyme site of an insert of pscFvCA-E8VHd and the amino acid sequence coded by the base sequence (1).
Fig. 8 is a diagram showing the restriction enzyme site of an insert of pscFvCA-E8VHd and the amino acid sequence coded by the base sequence (2).
Fig. 9 is a diagram schematically showing a structure of vector scNcopFCAH9-E8VHdVLd used for the preparation of scFv-CL antibody gene library.
Fig. 10 is a diagram showing the base sequence of an insert section of scNcopFCAH9-E8VHdVLd and the amino acid sequence coded by the relevant base sequence (1).
Fig. 11 is a diagram showing the base sequence of an insert section of scNcopFCAH9 -E8VHdVLd and the amino acid sequence coded by the relevant base sequence (2).
Fig. 12 is a diagram showing the base sequence of an insert section of scNcopFCAH9 -E8VHdVLd and the amino acid sequence coded by the relevant base sequence (3).

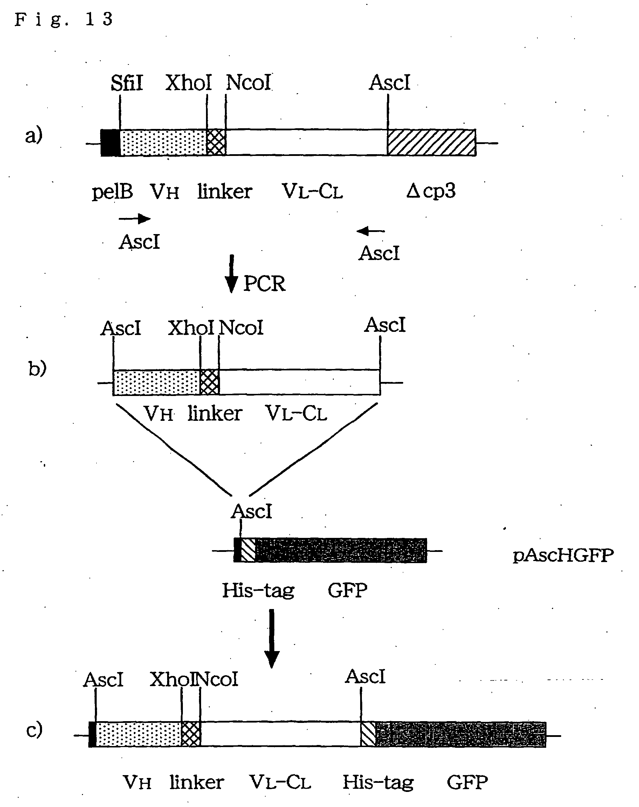

Fig. 13 is a diagram showing an operational procedure in Example of the present invention. Diagram shown in a) is a schematic diagram of gene of cp3 fusion type scFv-CL antibody in a clone in AIMS-5 library. Diagram shown in b) is a schematic diagram showing that DNA of region where scFv-CL antibody is coded is amplified by PCR and the PCR product is incorporated into pAscHGFP vector. The PCR Product is shown in the upper portion of b) (amplified scFv-CL antibody gene), pAscHGFP vector is shown in the lower portion. Diagram shown inc) is a vector for expressing GFP fusion type scFv-CL antibody, which is obtained by the operation of b).
Fig. 14 is a diagram showing the DNA sequence and amino acid sequence of scFv(CC046N2)-CL-GFP. It is a structure in which it is ordered as VH domain-linker-Vλ domain-Cλ domain -His-tag-GFP following 3-amino acids containing methionine. The number of V domain and the location of CDR were determined according to the determination method of Kabat. Amino acid located at the tenth of Vλ domain is deleted.
Fig. 15 is a diagram showing the results (gel) that GFP fusion type scFv-CL antibody which had been expressed using *E. coli* was analyzed by SDS-PAGE. Lane M is the result that molecular weight marker was flowed, lane I is the result that crude extract was flowed, and lane 2 is the result that the purified protein was flowed. The straining was carried out with Coomassie brilliant blue. The arrow indicates the location of GFP fusion type scFv-CL antibody.

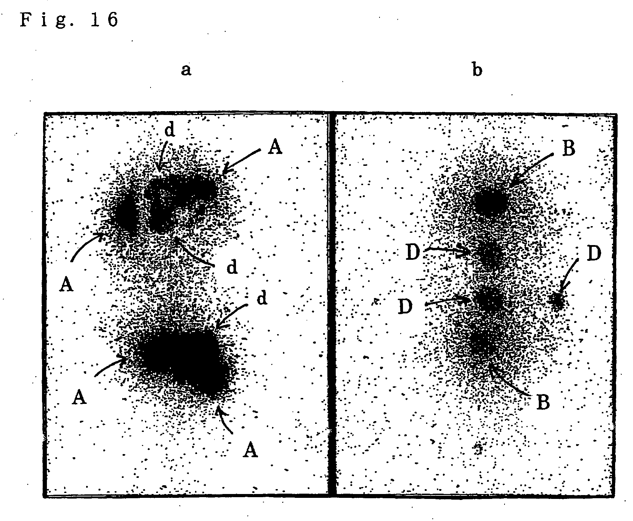

Fig. 16 is a diagram showing that an early embryo of C. elegans was stained with scFv-CL antibodies in forms of two kinds specific to CC046. Diagram a) is a diagram showing that it was directly stained with GFP fusion type scF-CL-GFP (Fluorescence of GFP is observed in the portion indicated by the arrow A. And, fluorescence by DAPI is observed in the portion indicated by the arrow d. Diagram b) is a diagram showing that after cp3 fusion type scFv-CL antibody was reacted, the secondary antibody (rabbit anti-cp3 antibody) and tertiary antibody (Cyanin 3 labeled goat anti-rabbit IgG antibody) were reacted and detected (Fluorescence of Cyanin 3 is observed at the portion indicated by the arrow B. And, fluorescence by DAPI is observed at the portion indicated by the arrow D).

### DESCRIPTION OF THE PREFERRED EMBODIMENTS

A method for preparing a scFv antibody with which a fluorescent protein has been fused, which is the present invention, comprising the following steps:
1) a step of preparing a scFv antibody library composed of phage clones, each one of which expresses a scFv antibody on the surface;
2) a step of selecting a phage clone expressing a scFv antibody capable of binding to the relevant antigen by screening the foregoing scFv antibody library with an antigen,
3) a step of acquiring the gene coding for the scFv antibody from the phage clone which has been selected in the Step 2);
4) a step of incorporating the relevant gene into an expression vector capable of expressing a fusion protein of the expression product of the relevant gene and a fluorescent protein by incorporating the gene acquired in the step 3); and
5) a step of transforming a host using the recombinant vector obtained in the step 4) and expressing the fusion protein.

In the present invention, "scFv antibody" is an antibody which contains a Fv region, that is, a heavy chain variable region (VH region) and a light chain variable region (VL region), and which is constructed by a cross-linkage of these two regions using a linker. In the present specification, it is also defined that a scFv antibody includes an antibody containing a light chain constant region (CL region) in addition to a VH region, a VL region and a linker. The Addition of CL region is carried out for the purpose of the stabilization of the conformation of a scFv antibody with which a fluorescent protein has been fused, which is the present invention.

Moreover, "scFv antibody gene" is composed of a gene coding for a VH region (VH gene), a gene coding for a VL region (VL gene) and a linker sequence. In the present invention, it is defined that a scFv antibody gene includes an antibody gene containing a gene coding for a CL region (CL gene) in addition to those.

As a linker which cross-links between a VH region and a VL region, a general-purposed linker can be used, for example, a peptide linker consisting of glycine-serine is used.

In the present invention, the term "library" means an assembly consisting of a variety of homologous components. Hence, scFv antibody library is an assembly containing a variety of scFv antibodies. In the present invention, the respective scFv antibodies are expressed on the surface of a phage. In other words, a scFv antibody library is formed with an assembly of phage clones expressing a scFv antibody on its surface.

A scFv antibody library will be effective if it could be prepared so that a heavy chain variable region and a light chain variable region reconstruct a functional conformation (or, so that most members of the reconstructed ones). The present inventors have proposed that a light chain variable region for forming a functional conformation with a heavy variable region are selected and the selected one is used for the preparation of a library in Japanese Patent Application No.12-050543. The method proposed in Japanese Patent Application No.12-050543 is also capable of being applied to a scFv antibody library. Moreover, it is preferable that a scFv antibody library has a sufficient number of clones to contain the intravital diversity. By satisfying this, it is possible to select a clone expressing a scFv antibody on its surface, which is capable of binding to the relevant antigen among various kinds of antigens in the step 2) described later. Moreover, it is preferable that the number of clones is 1 × 10¹¹ or more. A method for preparing a scFv antibody library having such a number of clones will be described in Example described later.

A scFv antibody library in the present invention is composed of the assembly of phage clones expressing a scFv antibody on its surface, such a scFV antibody library can be prepared from the assembly of phagemid clones holding a scFv antibody gene (hereinafter, referred to as "scFv antibody phagemid library"). It should be noted that in the present invention, a library composed of the assembly of clones holding a scFv antibody gene is referred to as a scFv antibody gene library. Therefore, a scFv antibody phagemid library is one of the scFv antibody gene libraries. Moreover, the assembly of E. coli transformed by a phagemid holding a scFv antibody gene also constitutes a scFv antibody gene library.

As a phagemid vector for preparing a scFv antibody phagemid library, those commercially available can be used (For example, pTZ19R; made by Pharmacia). In a phagemid, a gene coding for a foreign protein desired to be expressed, that is, a scFv antibody gene is connected to a gene coding for constitutive protein of phage such as cp3, cp8 or the like.

As described above, a scFv antibody gene which consists of a VH gene, a VL gene and a linker sequence or a sequence in which a CL gene is added to these, a VH gene, a VL gene and a CL gene can be obtained from an optional antibody production cell. As an antibody production cell, for example, peripheral blood lymphocyte, spleen cell and the like can be listed. For the isolation of the respective genes, a RT-PCR method using a known primer can be utilized. A scFv antibody gene is constructed by connecting the obtained genes in the respective regions and a linker sequence.

A method for preparing a scFv antibody library from a scFv antibody phagemid library can be carried out as follows: first, a host is transfected with the respective clones constituting a scFv antibody phagemid library. Next, the host is superinfected with a helper phage. For example, phagemid vector pTZ19R can be recovered as a phage particle by superinfection with helper phage M13K07.If cp3 protein of phagemid utilized at this time is fused with a foreign protein, that is, a scFv antibody, the scFv antibody is displayed on the surface of the phage to be completed.

A method for preparing a scFv antibody library is not limited to the methods described above, for example, as in Examples described later, first, a library composed of clones, each one of which holds a Fab type antibody gene, which consists of a VH gene, a CH gene, a VL gene and a CL gene(hereinafter, referred to as "Fab antibody gene library"), is prepared, and the assembly of clones, each one of which holds a scFv antibody (scFv antibody gene library), is obtained by converting a Fab antibody gene in the above respective clones into a scFv antibody gene, and from this assembly, a scFv antibody library can be prepared by the method described above.

In the step 2) of the present invention, a scFv antibody library is screened with the specific antigen, and a phage clone expressing a scFv antibody capable of binding to the relevant antigen is selected.

As for an antigen, a variety of antigens are used according to the purposes. Moreover, the screening using an antigen can be carried out by a panning method indicated below. First, an antigen of interest is made contacted with a scFv antibody library, and a clone binding to this antigen is recovered. The clone recovered is amplified, and then the step in which the amplified clones are contacted again with the antigen of interest, and the clone capable of binding to its antigen is recovered is repeated. The amplification of a phage is carried out by infecting the phage with *E. coli* and the recovering it. The phage clone expressing a scFv antibody having the reactivity of interest on its surface is condensed by repeating this step. It is general that the screening based on the binding characteristic with an antigen is carried out until the collection ratio is raised. The collection ratio is a ratio of the number of phage clones recovered as one having the binding characteristic to the antigen with respect to the number of phage clones added to the antigen. When the recovery ratio is clearly raised comparing to the last screening, it means that a phage clone expressing a scFv antibody having the reactivity of interest on its surface is getting condensed.

In the step 3), the gene coding for a scFv antibody (scFv antibody gene) is acquired from the phage clone selected by the step 2). Specifically, the gene coding for a VH region, a VL region and a linker is cut out from the phage clone. In the case where as a scFv antibody, an antibody containing a CL region is used, a gene coding for a CL region in addition to a VH region, a VL region and a linker is cut out. At this time, an antibody containing Tag such as His, Myc and the like may be used.

As a concrete method for cutting out a scFv antibody gene, a restriction enzyme which specifically cuts a scFv antibody gene introduction sites of a phage DNA, that is, the restriction enzyme sites located at both ends of a scFv antibody gene is used. In order to carry out such an cutting out, a scFv gene introduction site in each phage clone in a scFv antibody library has been previously formed as a unique restriction enzyme site corresponding to the relevant restriction enzyme.

Following the step 3), the step (step 3-1) in which sequences located at one end or both ends of a scFv antibody gene acquired in the step 3) is converted into a different base sequence under the conditions where an amino acid sequence coded by the relevant gene is not changed can be carried out. By this step, the desired restriction enzyme site can be formed at one end or both ends without changing the amino acid sequence coded by a scFv antibody gene. Owing to this, the base sequence of one end or both ends of the scFv antibody gene cut in the step 3) can be corresponded to the restriction enzyme sites which forms the introduction site of the expression vector used in the next step 4). The degree of freedom of the design for the expression vector will be enhanced by carrying out such a step. Needless to say, a scFv antibody gene and an expression vector can be previously designed so that the restriction enzyme sites of both ends of the scFv antibody gene and the introduction site of the expression vector are corresponded each other, in this case, the step 3-1) is not required. The step 3-1) can be carried out by PCR using an appropriate synthetic primer. In this case, the step 3-1) can be carried out with the step 3) at the same time. Specifically, when a scFv antibody gene is cut out, PCR is carried out by utilizing two primers for converting sequence of one end or sequences of both ends of a scFv antibody gene, the converted scFv antibody gene of the restriction enzyme site is cut out and amplified.

Moreover, the desired restriction enzyme site may be formed by adding an appropriate linker to one end or both ends of scFv antibody gene.

In the step 4), the incorporation of the scFv antibody gene acquired in the step 3), or the step 3) and the step 3-1) into the expression vector is carried out.

As for an expression vector, a vector that is capable of expressing a fusion protein of a scFv antibody gene expression product and a fluorescent protein by incorporating a scFv antibody is used. As such an expression vector, a vector having, in turn starting from the 5' end side, a start codon, a site into which the scFv antibody gene is introduced, and the base sequence coding for a fluorescent protein is used. Moreover, an expression vector having the base sequence coding for His-tag, myc-tag, HA-tag or the like between a site into which the scFv antibody gene is introduced and the base sequence coding for a fluorescent protein [(hereinafter, referred to as "tag sequence") can be used. By using such a vector, a molecule coded by a tag sequence which intervens between a scFv region and a fluorescent protein. Owing to this, the purification of the scFv antibody is capable of being carried out by utilizing the affinity of a tag molecule for the specific substance. Moreover, the location into which the tag sequence is introduced is not limited, it can be introduced into the upstream region of the 5' end of the scFv antibody gene introduction site or into the downstream region of the sequence coding for a fluorescent protein. However, it is necessary to introduce it into the location where it is expressed as a fused one of the molecule coded by the tag sequence and the scFv antibody.

An expression vector having the above-described characteristics can be prepared by altering commercially available expression vectors using a well-known genetic manipulation technique. It should be noted that a vector for bacteria expression, a vector for animal cell expression are used corresponding to the host to be transformed. As an expression vector in the present invention in the case where *E. coli* is made host, for example, a vector in which an AscI site is added between a start codon and a His-Tag sequence based on p6 × His-GFP (made by Clontech) vector can be used.

In this expression vector, a scFv antibody gene is introduced at AscI site. In the case where this vector is used, in the expression product obtained in the step 5) described later, the scFv antibody with which a histidine tag, that is, a labeling peptide, has been fused is expressed. Since the histidine tag is a tag having a binding activity with a metal ion, for example, since it can be trapped on a Nickel column, it can be utilized for the purification of a scFv antibody.

For the similar purpose, instead of histidine, myc-tag , HA-tag or the like can be used. In this case, similar to the case of histidine tag, an expression vector into which a base sequence coding for a tag listed above is incorporated is used. Moreover, previously the base sequence coding for His-tag or the like has been fused with the scFv antibody gene, and this can be introduced into a expression vector. In this case, as an expression vector, it is not necessary to use a vector having a base sequence coding for His-tag or the like.

The kinds of fluorescent proteins are not particularly limited, a known one can be optionally selected and used. For example, GFP, RFP, BFP, YFP and CFP can be used, and the mutants thereof can be also used. As a mutant, for example, EGFP (enhanced green fluorescent protein), EBFP (enhanced blue fluorescent protein), EYFP (enhanced yellow fluorescent protein) are known. These proteins can emit the light having a wavelength different from the irradiating light by irradiating the light having the specific wavelength, and these can be optionally selected and used according to the purpose, conditions and the like. Moreover, needless to say, it is not limited to the cases where only one type of these fluorescent proteins is used, two or more types can be optionally selected, combined and used.

The present invention includes a recombinant vector having a scFv antibody gene and a base sequence coding for a fluorescent protein acquired as a result of the above-described steps 1) -4). Moreover, the present invention also includes a recombinant vector acquired by further carrying out the above-described step 3-1) following the step 3). Also in this case, as described above, the present invention includes a recombinant vector as a scFv antibody gene including a CL gene.

As a concrete recombinant vector, a vector having the following constitution can be listed.

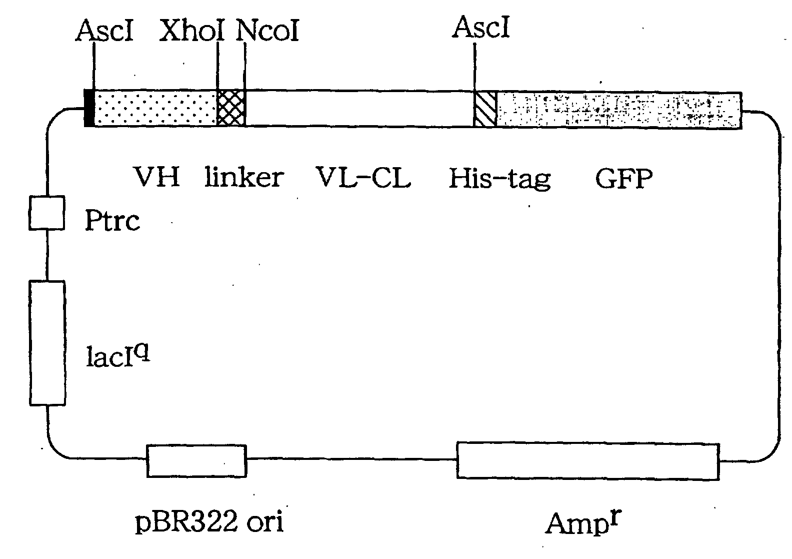

In the step 5) in the present invention, a host is transformed with a recombinant vector having a scFv antibody gene, which have been acquired in the step 4), and then the scFv antibody with which a fluorescent protein has been fused is expressed.

The scFv antibody fused with a fluorescent protein, which has been prepared by the above-described steps, can be utilized for an immunological measurement method such as immunostaining or the like. In a scFv antibody of the present invention, since a fluorescent protein is fused, the scFv antibody can emit the fluorescence by itself. Specifically, in an ELISA method or the like using the conventional antibody, the labeled secondary antibody is required, however, in a scFv antibody of the present invention, since the scFv antibody itself has been labeled, a secondary antibody or the like is not required to use, and the simplification of the measuring steps is achieved.

Moreover, since a scFV antibody can emit the fluorescence by itself, a special substrate is not required, for example, the measurement can be directly carried out by expressing within the cell without taking out the antibody from the cell. Therefore, the expression and distribution of the specific antigen in the cell or in vivo can be directly measured.

A kit for preparing a scFv antibody can be constituted by containing a scFc antibody library composed of phage clones expressing a scFv antibody on its surface and a vector which is, by incorporating a scFv gene, capable of expressing a fusion protein of the relevant gene expression product and a fluorescent protein. As a scFv antibody library, a fluorescent protein and an expression vector, the above-described ones can be optionally selected and used. Moreover, as a scFv antibody, a scFv antibody consisting of a VH region, a VL region, a linker and a CL region is also included.

Moreover, a kit for preparing a scFv antibody with which a fluorescent protein has been fused can be constituted by including a scFv antibody gene library consisting of phage clones having a scFv antibody gene or phagemid clones having a scFv antibody gene and a vector which is, by incorporating a scFv gene, capable of expressing a fusion protein of the expression product of the relevant gene and the fluorescent protein. In this case, a scFv antibody gene can be composed of a VH gene, a VL gene, a linker sequence and a CL sequence. A scFv antibody gene library is prepared by introducing a scFv antibody gene using the known phage or phagemid and by means of the known gene operation technique. Moreover, as a fluorescent protein and an expression vector, the above-described ones can be optionally selected and used.

### [Example]

In the present Example, in order to prepare a scFv antibody library, first, a Fab antibody gene library comprising the assembly of clones containing a Fab type antibody gene which consists of a VH region, a CH region, a VL region and a CL region was prepared. Then, the Fab antibody gene library was converted into a scFv antibody gene library comprising the assembly of clones containing a scFv type antibody gene, thereby preparing a scFv antibody library. It should be noted that in the present Example, as a scFv type antibody, an antibody in which a CL region has been added to a VH region, a linker and a VL region (scFv) (hereinafter, referred to as "scFv-CL antibody") was used. The reason why the CL region has been added is the promotion of the stability in the case where the antibodies are expressed within the cytoplasm is expected. It should be noted that a method for preparing a scFv antibody library is not limited to the following one.

### 1. Preparation of phagemid vector for preparing Fab antibody gene library

### 1-1 Preparation of vector for preparing Fab antibody gene library

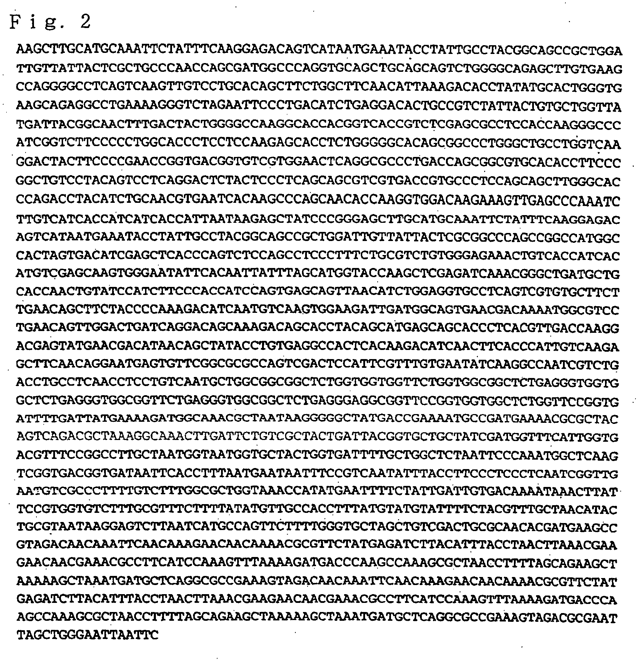

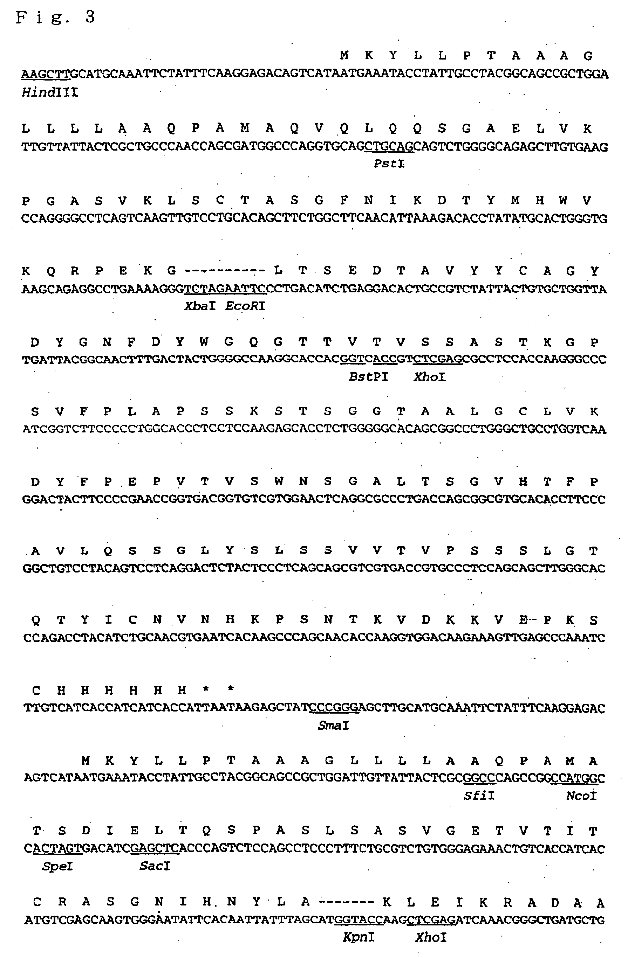

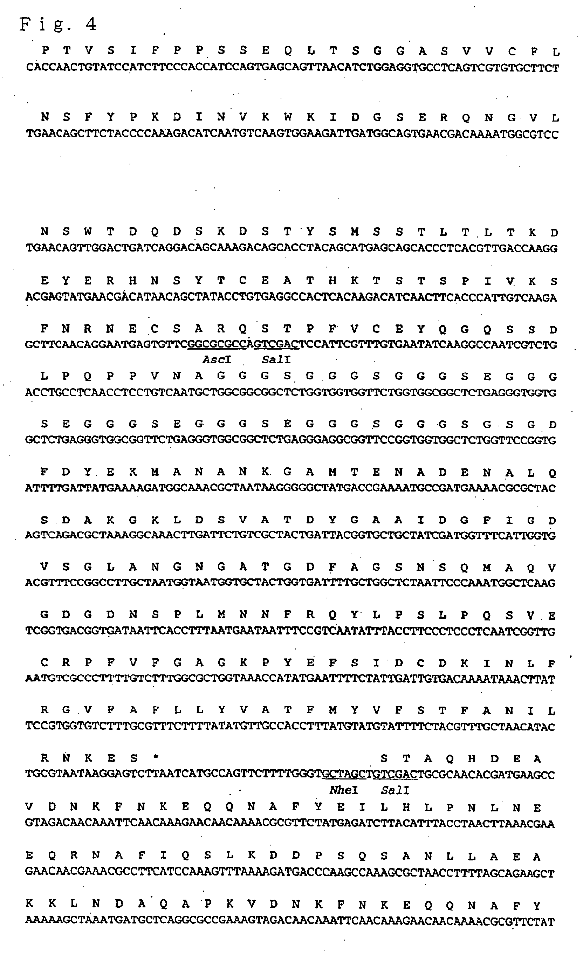

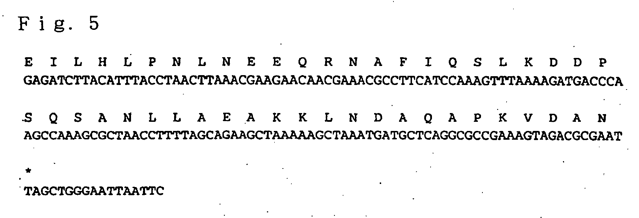

As conceptually shown in Fig. 1, a pFCAH9-E8d vector was prepared by incorporating pelB (signal sequence) of M13 phage, His 6 tag sequence, cp3 protein of M13 phage (Δcp3(198aa-406aa) N-end deleted capsid protein 3) sequence, and protein A protein sequence in pTZ19R phagemid vector (Pharmacia) at an appropriate restriction enzyme site (see Iba, Y. et al., Gene 194: 35-46, 1997). In order to avoid that the gene is cut at BstPI existing in a light chain λ5, λ6 gene, XhoI site has been introduced in pFCAH9-E8d. The base sequence of an insert of pFCAH9-E8d is shown in Fig. 2, the restriction enzyme site and the amino acid sequence coded by the base sequence are shown in Figs.3-5.

An actual antibody protein expression vector is to be completed by inserting a heavy chain and a light chain genes at the predetermined locations of this vector. The shape of the antibody expressed by the completed vector is in a Fab type, the heavy chain and light chain have constant regions CH1, CL following a variable region of N-terminal, respectively. The heavy chain and the light chain are to be bound by -SS- binding between constant regions. The light chain constant region CL gene has been bound to the above-described cp3 gene, as a result, the expression protein is to be in a shape of Fab-cp3.

Concretely, the following operations were carried out: Primer which has been used:

527 Reverse (SEQ ID NO.: 2) :
5'-CAGGAAACAGCTATGAC-3'

599 E8VHf-PstR (SEQ ID NO.: 3) :

3'-CGGCTCCAAGTCGACGTCGTCA-5'

544 E8VHf-PstF (SEQ ID NO.: 4) :

5'-CAGCTGCAGCAGTCTGGGGCAGAGCTTGTGAAGCCAGGGGCCTCAGTCAAGTTGTC

CTGCACAGCTTCTGGCTTCAACATTAA-3'

545 E8VHf-XbaR (SEQ ID NO.: 5) :

3'-AGACCGAAGTTGTAATTTCTGTGGATATACGTGACCCACTTCGTCTCCGGACTTTT

CCCAGATCTCACCTAACCTTCCTAA-5'

546 E8VHf-XbaF (SEQ ID NO.: 6) :

5'-AAGGGTCTAGAGTGGATTGGAAGGATTGATCCTGCGAGTGGTAATACTAAATATGA

CCCGAAGGACAAGGCCACTATAACAGCA-3'

547 E8VHf-EcoR (SEQ ID NO.: 7) :

3'-TTCCTGTTCCGGTGATATTGTCGTCTGTGTAGGAGGTTGTGTCGGATGGATGTCGA

CTTAAGGGAC-5'

548 E8VHf-EcoF (SEQ ID NO.: 8) :

5'-CAGCTGAATTCCCTGACATCTGAGGACACTGCCGTCTATTACTGTGCTGGT-3'

549 E8VHf-BstR (SEQ ID NO.: 9) :

3'-CAGATAATGACACGACCAATACTAATGCCGTTGAAACTGATGACCCCGGTTCCGTG

GTGCCAGTGGCACAAGG-5'

590 His6-SmaR (SEQ ID NO.: 10) :

3'-GGTTCTCTAACAGTAGTGGTAGTAGTGGTAATTATTCTCGATAGGGCCCTCGAA-5

'

542 E8VLf-SacF (SEQ ID NO.: 11) :

5'-GACATCGAGCTCACCCAGTCTCCAGCCTCCCTTTCTGCGTCTGTGGGAGAAACTGT

CACCATCACATGT-3'

539 E8VLf-KpnR (SEQ ID NO.: 12) :

3'-TGACAGTGGTAGTGTACAGCTCGTTCACCCTTATAAGTGTTAATAAATCGTACCAT

GGTCGTC-5'

542 E8VLf-KpnF (SEQ ID NO.: 13) :

5'-GCATGGTACCAGCAGAAACCAGGGAAATCTCCTCAGCTCCTGGTCTAT-3'

543 E8VLf-BamR (SEQ ID NO.: 14) :

3'-GGAGTCGAGGACCAGATATTACGTTTTTGGAATCGTCTACCACACGGTAGTTCCAA

GTCACCGTCACCTAGGCCTTGTGTT-5'

562 E8VLf-XhoR (SEQ ID NO.: 15) :

3'-TCATGAGGCACCTGCAAGCCACCTCCGTGGTTCGAGCTCTAGTTT-5'

563 E8VLf-XhoF (SEQ ID NO.: 16) :

5'-AGTACTCCGTGGACGTTCGGTGGAGGCACCAAGCTCGAGATCAAA-3'

613 NheR (SEQ ID NO.: 17) :

3'-ATCGACAGCT-5'

600 E8VLKpnXhoR (SEQ ID NO.: 18) :
3'-AAGCCACCTCCATGGTTCGAGCTCTAGTTT-5'

LCP3ASC (SEQ ID NO.: 19) :

3'-TCGAAGTTGTCCTTACTCACAAGCCGCGCGGTCAGCTGAGGTAA-5'

hCH1Bst (SEQ ID NO.: 20) :
5'-ACCCTGGTCACCGTCTCCTCAGCCTCCACCAAGGGCCCATCGGTCTTCCCCCTGG-
3'

hCH1midAS (SEQ ID NO.: 21) :

3'-GGGAGTCGTCGCAGCACTGGCACGGAGGTCGTCGAA-5'

hCH1midS (SEQ ID NO.: 22) :
5'-GGACTCTACTCCCTCAGCAGCGTCGTGACCGTGCCC-3'

hCH1H6 (SEQ ID NO.: 23) :

3'-GGGTCGTTGTGGTTCCACCTGTTCTTTCAACTCGGGTTTAGAACAGTAGTGGTAGT

AGTGGTA-5'

hCH1H6Sma (SEQ ID NO.: 24) :

3'-GGGTTTAGAACAGTAGTGGTAGTAGTGGTAATTATTCTCGATAGGGCCCTCGAACG-5'

702 BstXhoF (SEQ ID NO.: 25) :
5'-GGCACCACGGTCACCGTCTCGAGCGCCTCCACC-3'

### Preparation of pFCAH3-E8T H chain portion

1) A DNA fragment was prepared by carrying out PCR using 527-599 and PCR using 547-590 by making pAALFab as a template.
2) A DNA fragment was prepared by carrying out PCR using 544-545, 546-547, 548-549.
3) 1) and 2) were mixed, PCR was carried out using 527, 590 and this was cloned into HindIII-SmaI site of pAALFab.
4) A DNA fragment was prepared by carrying out PCR using 542-562, 561-613.
   pFCAH3-E8T L chain portion
5) A DNA fragment was prepared by carrying out PCR using 538-539, 542-543.
6) 4) and 5) were mixed, PCR was carried out using 538, 562 and this was cloned into SacI-NheI site of pAALFab.
   pFCAH9-E8d
6) Preparation of VH stuffer portion
   After pFCAH3-E8T was digested with XbaI, EcoRI and changed into a blunt end by being acted with a klenow fragment, self ligation was carried out, thereby preparing a stuffer of a VH portion.
7) Preparation of VH stuffer portion
   PCR was carried out using 527-600 by making pFCAH3-E8T as a template, and cloned into HindIII-XhoI site of 6).
8) This was digested with KpnI, the self ligation was carried out and a stuffer of the VL portion was prepared.
9) Introduction of SfiI, NcoI, SpeI sites
   PCR was carried out using 527-663 by making pFCAH3-E8T as a template, and cloned into HindIII-SacI site of 1).
10) Introduction of AscI site
   PCR was carried out using 527-LCP3ASC by making pFCAH3-E8T as a template, this was cloned into 2) by completely digesting it with SacI and partially digesting it with SalI.
11) Conversion of ΥCH1 portion into human gene
   Since BstPI site exists in the human γCH1 portion, cloning was carried out by designing this being deleted. By making tonsil cDNA as a template, after PCR was carried out using hCH1Bst-hCH1midS, hCH1midAS-hCH1H6, these were mixed, then PCR was carried out using hCH1Bst-hCH16Sma, and its DNA fragment was cloned into BstPI-Sma site of 3).
12) Introduction of Xho site
   By making 11) as a template, PCR was carried out using 702-663, this was cloned into BstPI-SacI site of 11).

### 1-2 Preparation of vector for temporarily cloning heavy chain variable region (VH)

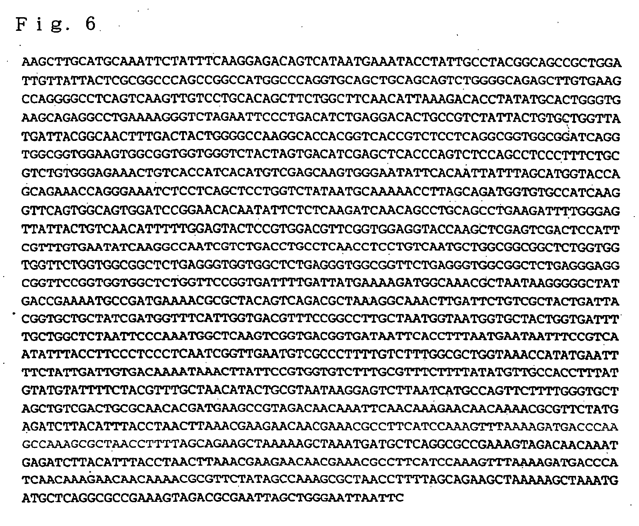

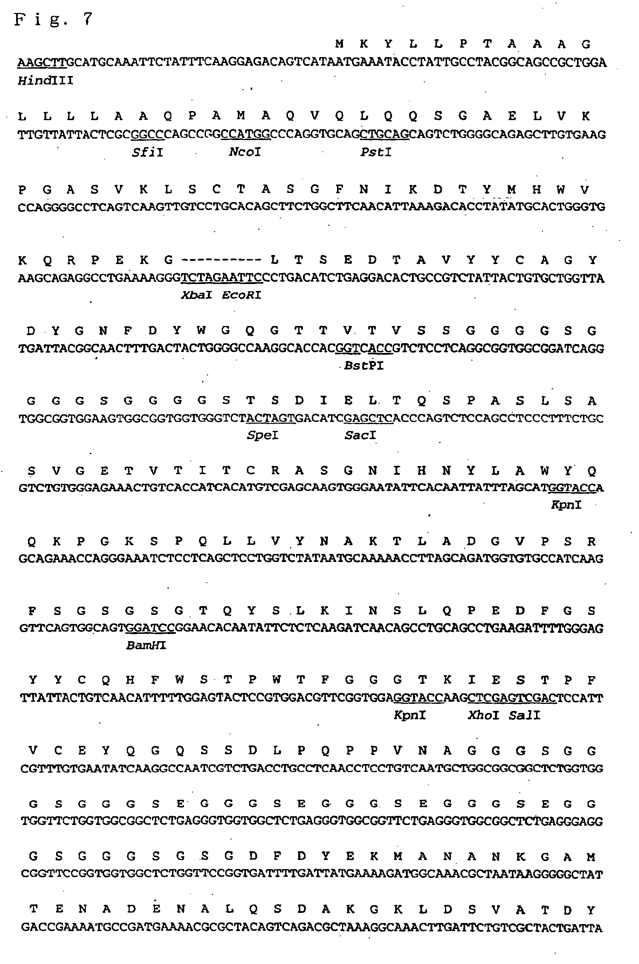

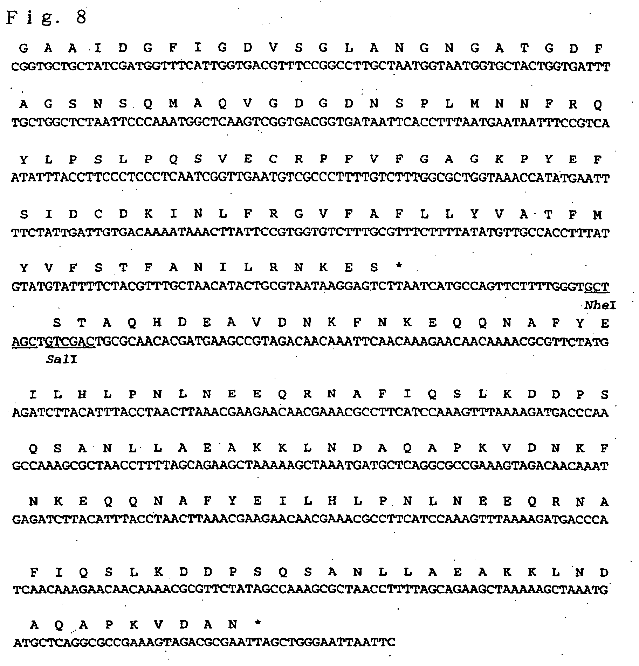

First, according to the known technique (see Iba, Y. et al., Gene 194: 35-46, 1997), pAALFab vector (1 of Fig. 1) was prepared. A pscFvCA-E8VHd vector (3 of Fig. 1) in which XbaI through EcoRI of pAALFab vector was deleted, restriction enzyme cut sites Kpn I, Sfi I, Nco I and Spe I were newly added, and a VH (heavy chain variable region) is capable of cloning via pFCAH3-E8T was prepared, and was made a vector for temporarily cloning a heavy chain variable region. The base sequence of an insert of pscFvCA-ESVHd is shown in Fig. 6, restriction enzyme sites and the amino acid sequence coded by and base sequence are shown in Figs.7-8.

Concretely,

610 scBstSpeSacF (SEQ ID NO.: 26)

5'-CACCACGGTCACCGTCTCCTCAGGCGGTGGCGGATCAGGTGGCGGTGGAAGTGGCG

GTGGTGGGTCTACTAGTGACATCGAGCTCACCCAG-3'

611 scBstSpeSacR (SEQ ID NO.: 27)

3'-GTGGTGCCAGTGGCAGAGGAGTCCGCCACCGCCTAGTCCACCGCCACCTTCACCGC

CACCACCCAGATGATCACTGTAGCTCGAGTGGGTC-5'

527 Reverse (SEQ ID NO.: 28)
5'-CAGGAAACAGCTATGAC-3'

619 E8VHf-SfiNcoPstR (SEQ ID NO.: 29):

3'-GACGCCGGGTCGGCCGGTACCGGCTCCAAGTCGACGTCGTCA-5'

Primer 610 and primer 611 were annealed, which was cloned into BstPI-SacI site of pFCAH3-E8T, thereby preparing a single chain. Furthermore, PCR was carried out using primer 527 and primer 629, which was further cloned into HindIII-PstI site, and the introduction of SfiI, NcoI sites are carried out.

### 2. Preparation of immunoglobulin light chain library

### 2-1 Isolation of immunoglobulin light chain gene using PCR

2.6µg of mRNA was obtained using commercially available kit (QuickPrep Micro mRNA purification kit; made by Pharmacia Biotech) from 4 × 10⁷ bone marrow cell (specimen No.59) and lymphocyte collected from umbilical blood and peripheral blood. A cDNA was prepared from this mRNA. The cDNA was prepared by Superscript Preamplification System made by GibcoBRL. An oligo dT was used for primer. By making the obtained cDNA as a template, PCR was carried out using 5'primer (κ1-κ6, λ1-λ6) and 3' primer (hCKASC primer or hCLASC primer) for acquiring a light chain gene. After phenol treatment, the PCR product was precipitated with ethanol and suspended in 10µL of TE buffer. The base sequence of primer employed and PCR conditions are as follows:
Noted that the underlined portions in the base sequences of primers for acquiring light chain gene indicate SfiI site, AscI site.

5'primer κ1-κ6

hVK1a (SEQ ID NO.: 30)

GTCCTCGCAACTGCGGCCCAGCCGGCCATGGCC GACATCCAGATGACCCAGTCTCC

hVK2a (SEQ ID NO.: 31)

GTCCTCGCAACTGCGGCCCAGCCGGCCATGGCC GATGTTGTGATGACTCAGTCTCC

hVK3a (SEQ ID NO.: 32)

GTCCTCGCAACTGCGGCCCAGCCGGCCATGGCC GAAATTGTGTTGACGCAGTCTCC

hVK4a (SEQ ID NO.: 33)

GTCCTCGCAACTGCGGCCCAGCCGGCCATGGCC GACATCGTGATGACCCAGTCTCC

hVK5a (SEQ ID NO.: 34)

GTCCTCGCAACTGCGGCCCAGCCGGCCATGGCC GAAACGACACTCACGCAGTCTCC

hVK6a (SEQ ID NO.: 35)

GTCCTCGCAACTGCGGCCCAGCCGGCCATGGCC GAAATTGTGCTGACTCAGTCTCC

5'primer κ1-κ6

hVL1 (SEQ ID NO.: 36)

GTCCTCGCAACTGCGGCCCAGCCGGCCATGGCC CAGTCTGTGTTGACGCAGCCGCC

hVL2 (SEQ ID NO.: 37)

GTCCTCGCAACTGCGGCCCAGCCGGCCATGGCC CAGTCTGCCCTGACTCAGCCTGC
hVK3a (SEQ ID NO.: 38)
GTCCTCGCAACTGCGGCCCAGCCGGCCATGGCC TCCTATGTGCTGACTCAGCCACC
hVL3b (SEQ ID NO.: 39)
GTCCTCGCAACTGCGGCCCAGCCGGCCATGGCC TCTTCTGAGCTGACTCAGGACCC
hVL4 (SEQ ID NO.: 40)
GTCCTCGCAACTGCGGCCCAGCCGGCCATGGCC CACGTTATACTGACTCAACCGCC
hVL5 (SEQ ID NO.: 41)
GTCCTCGCAACTGCGGCCCAGCCGGCCATGGCC CAGGCTGTGCTCACTCAGCCGCC
hVL6 (SEQ ID NO.: 42)
GTCCTCGCAACTGCGGCCCAGCCGGCCATGGCC AATTTTATGCTGACTCAGCCCCA
3'primer hCKASC (SEQ ID NO.: 43)
TCGACTGGCGCGCCGAACACTCTCCCCTGTTGAAGCTCTTTGTG
3'primer HCLASC (SEQ ID NO.: 44)
TCGACTGGCGCGCCGAACATTCTGTAGGGGCCACTGTCTTCTC

| Conditions of PCR | |
|---|---|
| CDNA | 2µL |
| 10 × buffer #1 (Attached to KOD) | 10µL |
| dNTP mix (2.0 mM) | 10µL |
| 25 mM MgCl₂ | 4µL |
| 5' end side primer (100 pmol/µL) | 1µL |
| 3' end side primer (100 pmol/µL) | 1µL |
| already sterilized MilliQ | 71µL |
| KOD DNA polymerase (2.5U/µL; made by Toyobo) | 1µL |

35 cycles of PCR carrying out one cycle at 94°C for one minutes, at 55°C for 2 minutes and at 74°C for one minute.

### 2-2 Method for preparing light chain gene library by selecting light chain suitable for preparation of library and

### 2-2-1 Incorporation of light chain gene into phagemid

PCR product obtained in 1 was treated with restriction enzyme under the following conditions:

| | |
|---|---|
| PCR product | 10µL |
| 10 × NEB4 (attached to AscI) | 5µL |
| 10 × BSA (attached to SfiI) | 5µL |
| Already sterilized MilliQ | 28µL |
| AscI (10 U/µL; made by NEB) | 1µL |
| SfiI (20 U/µL; made by NEB) | 1 µL |

After reacting at 37°C for one hour and at 50°C for one hour, the portion of 10µL out of it was subjected to agarose electrophoresis, the band nearby 600bp was cut out, purified by Gene Clean II Kit (made by Funakoshi). pFCAH9-E8d, which had been treated with restriction enzyme in the same manner as PCR product, was purified with Gene clean II kit and then ligated by reacting it with PCR product treated by restriction enzyme at 16°C for 4 hours to over night under the following conditions:

| | |
|---|---|
| pFCAH9-E8d treated with restriction enzyme | 2µL |
| PCR product treated with restriction enzyme | 1µL |
| 10 × ligation buffer | 1.5µL |
| (Attached to T4 DNA ligase) | |
| 10 mM ATP | 1.5 µL |
| sterilized MilliQ | 8µL |
| T4 DNA ligase (10 U/µL; made by Takara) | 1 µL |

### 2-2-2 Introduction of phagemid into E. coil

E. coli DH12S was transformed using the obtained ligated DNA as follows:

Specifically, ligated DNA was once precipitated with ethanol, and dissolved in 3µL of 1/5TE (TE diluted 5-fold with sterilized MilliQ). Out of it, 1.5µL was suspended in 20µL of competent cell DH12S (made by GIBCO BRL), the electroporation was carried out under the following conditions:

### Electroporator

Cell-Porator (Cat. series 1600) made by BRL

| | |
|---|---|
| Setting conditions: voltage booster | 4 kΩ |
| Capacitance | 330µF |
| DC volts | Low Ω |
| Charge rate | Fast |

### 2-2-3 Secretion of Fab-cp3 type antibody from E. coli transformed with phagemid into medium

The above-described transformed E. coli was inputted in 2 mL of the medium for transformation (SOB), after shaken and cultured at 37°C for one hour, one portion of it plated on the agar medium (Amp plate). the remains were cultured in 1% glucose, 100µg/mL ampicilin containing 2 × TY medium and stocked with glycerin. The agar medium was incubated at 30°C, the growing colony was separated by picking at it with a stick, a plasmid was prepared, respectively, and the base sequence of the light chain gene was examined.
SOB medium: the following components were added to 950 mL of the purified water and shaken, after completely dissolved, 10 mL of KCl solution in 250 mM was added, and adjusted at pH 7.0 with 5N NaOH. After the purified water was added to adjust the volume into 1000 mL, it was sterilized using autoclave for 20 minutes, and 5 mL of MgCl₂ in 2M sterilized immediately before the use was added.

| | |
|---|---|
| Bacto-tryptone | 20g |
| Bacto-yeast extract | 5g |
| NaCl | 0.5g |

2 × YT medium: the following components were added to 900 mL of the purified water and shaken, after completely dissolved, it was adjusted at pH 7.0 with 5N NaOH, the purified water was added to make the volume into 1000 mL. Sterilized by autoclave for 20 minutes, and used.

| | |
|---|---|
| Bacto-tryptone | 16g |
| Bacto-yeast extract | 10g |
| NaCl | 5g |

The other reagents were purchased from the followings:

| | |
|---|---|
| Maker | Name of product |
| Sigma | Ampicillin sodium |
| Wako Junyaku | Phenol |
| Sigma | BSA |
| DIFCO | 2 × YT medium |
| Wako Junyaku | kanamycin sulfate |
| Nacalai tesque polyethylene glycol | 6000 |
| Nacalai tesque Tween | 20 |
| Katayama Chemical | NaCl |
| Wako Junyaku | IPTG |
| Wako Junyaku | skimmed milk |
| Wako Junyaku | sodium azide |
| Wako Junyaku | triethylamine |
| Wako Junyaku | hydrogen peroxide |
| Wako Junyaku | OPD tablet |
| Wako Junyaku | ethanol |

The above-described operations were carried out with respect to all of κ1, κ2, κ3, κ4, κ5 and κ6, and λ1, λ2, λ3a, λ3b, λ4, λ5, λ6, λ7, λ8, λ9 and λ10, and whether or not the clone of interest was obtained was confirmed. Subsequently, clones of the respective groups such as κ1, κ2 and the like were mixed so as to become nearby ratio of the usage frequency in vivo. As for the groups of these light chains, it was already known at what ratio these actually express in vivo. These gene clones amplified by PCR and incorporated in a vector were mixed so as to become at a ratio nearby the usage frequency in vivo, and made VL library. The constitutive ratio of the respective families in VL library is indicated as follows:

| Vκ | | | |
|---|---|---|---|
| family | Usage frequency in vivo(%)* | Constitutive ratio in VL library (%) | Constitutive ratio in KL200 (%) |
| Vκ1 | 39 | 37 | 30.7 |
| Vκ2 | 12 | 12 | 19.8 |
| Vκ3 | 36 | 35 | 33.7 |
| Vκ4 | 12 | 12 | 10.9 |
| Vκ5 | 1 | 2 | 5.0 |
| Vκ6 | -** | 2*** | 1.0 |

| | | | |
|---|---|---|---|
| * Griffith AD et al. EMBO J. (1994) 13, 3245-60. | | | |
| ** No description when published. | | | |
| *** Equal quantities of cDNA prepared with primer VK6-2 and cDNA prepared with primer VK6-3 were mixed. | | | |

| Vλ | | | |
|---|---|---|---|
| family | Usage frequency in vivo(%)^{*} | Constitutive ratio in VL library (%) | Constitutive ratio in KL200 (%) |
| Vλ1 | 43 | 41 | 34.1 |
| Vλ2 | 15 | 15*³ | 15.2 |
| Vλ3 | 34 | 32*⁴ | 25.3 |
| Vλ4 | 0 | 1.5*⁵ | 0.0 |
| Vλ5 | 0 | 1.0*⁶ | 11.1 |
| Vλ6 | 0 | 1.0 | 14.1 |
| Vλ7 | 6 | 6 | 0.0 |
| Vλ8 | 1 | 1 | 0.0 |
| Vλ9 | 1 | 1 | 0.0 |
| Vλ10 | -*² | 1 | 0.0 |

| | | | |
|---|---|---|---|
| * Griffith AD et al. EMBO J. (1994) 13, 3245-60. | | | |
| *2 No description when published. | | | |
| *3 5% of cDNA prepared with primer VL2 and 10% of cDNA prepared with primer VL2-2 were mixed. | | | |
| *4 17% of cDNA prepared with primer VL3a-2 and 15% of cDNA prepared with primer VL3b were mixed. | | | |
| *5 0.5% of cDNA prepared with primer VL4a and 0.5% of cDNA prepared with primer VL4b and 0.5% of cDN prepared with VL4c were mixed. | | | |
| *6 0.5% of cDNA prepared with primer VL5abde and 0.5% of cDNA prepared with primer VL5c were mixed. | | | |

Next, about 1000 pieces of base sequences of light chain genes selected randomly from VL library was confirmed. Specifically, the base sequences were determined using fluorescent primer huCH1 (5'-ATTAATAAGAGCTATCCCGG-3'/SEQ ID No.:45) by a dideoxy method using thermosequence kit (made by Amersham Pharmacia) and L1-COR4200L(S)-2 (made by Aloka). The obtained base sequences were compared and overlapped clones were removed. Furthermore, made reference to data base, the clones confirmed that no deletion exist were combined with VH3-4 which is one of heavy chain genes and its expression was previously understood and an expression experiment was carried out. The operation was as follows: Note that amino acid sequence of VH3-4 is indicated as SEQ ID No.:1.

First, VH3-4 was digested with HindIII and XhoI, the heavy chain gene was cut out, and purified by Gene Clean II Kit. On the other hand, the light chain gene clone confirmed that no deletion exists was also digested with HindIII and XhoI, the light chain gene was cut out, purified by Gene Clean II Kit, and combined with the heavy chain gene of VH3-4 by carrying out the ligatiqn. Using the obtained ligated DNA, E. coli DH12S was transformed. The grown colony was inputted in the medium in a test tube, Fab-cp3 type antibody molecules were expressed in the supernatant in the culture medium by inducing the expression with IPTG. Fab-cp3 type antibody molecules were expressed in the culture supernatant even without helper phage infection by culture for about 20 hours. ELISA method was carried out using this culture supernatant as follows:

### 2-2-4 Test of correct expression and association of heavy chain an light chain by ELISA method

### 1) Preparation of antibody binding 96 well microtiter plate

Anti-K antibody (MBL code No.161) was diluted with 0.01M sodium-phosphate buffer pH 8.0 and 0.1% NaN₃, into 1.25µg/mL, 100µl each was added to a microtiter plate. By standing over night at 4°C, the anti-κ antibody was absorbed in a well. The reaction liquid was discarded, 200µL each of 5% BSA in 0.01M sodium-phosphate buffer pH 8.0, and 0.1% NaN₃ was added to the microtiter plate, and by standing at 37°C for 2 hours, the blocking for preventing the non-specific absorption was carried out.

Next, the anti-λ antibody (MBL code No.159) whose non-specific activity has already been absorbed was diluted with 0.01M sodium-phosphate buffer pH 8.0, 0. 1%NaN₃ into 2. 5µg/mL, 100µL each was added to the microtiter plate, and stood over night at ice room. The reaction liquid was discarded, 200µL each of 5% BAS in 0.01M sodium-phosphate buffer pH 8.0, and 0.1% NaN₃ was added to the microtiter plate, stood at 37°C for 2 hours, thereby the blocking for preventing the non-specific binding was carried out.

### 2) Primary reaction

As a positive control, 100µL each of human Fab(10µg/mL) was added, and as a negative control, 100µL each of PBS/0. 1%NaN₃ was added to the microtiter plate. 100µL each of the stock solution of the culture supernatant into which the expression of Fab-cp3 type antibody molcule has been induced by IPTG was added to the microtiter plate, and reacted at 37°C for one hour.

### 3) Secondary reaction

The microtiter plate which has terminated the primary reaction was washed with 0.05% Tween 20-PBS 5 times. Subsequently, 100µL each of the anti-Fd antibody (1µg/mL) diluted with PBS/0.1%NaN₃ was added to the microtiter plate, and reacted at 37°C for one hour.

### 4) Tertiary reaction

The microtiter plate which has terminated the secondary reaction was washed with 0.05% Tween 20-PBS 5 times. Subsequently, 100µL each of alkali phosphatase labeled anti-sheep IgG antibody (4000-fold dilution) diluted with PBS/0.1%NaN₃ was added to the microtiter plate, and reacted at 37°C for one hour.

### 5) Coloring reaction and measurement of absorbance

The microtiter plate which has terminated the tertiary reaction was washed with 0.05% Tween 20-PBS 5 times. Subsequently, 100µL each of coloring substrate solution (SIGMA 104 phosphatase substrate tablets; each tablet dissolved in 5mL of 50mM diethanol amine pH 9.8) was added to the microtiter plate. Reacted in room temperature, at the time when the 405nm of absorbance might be 0.5 or more, stop solution was added, and the absorbance was measured using plate reader (Titertech Multiscan MCC).

The clone which was positive (absorbance: 0.5 or more) by the ELISA method was determined that the expression and association of Fab-cp3 type antibody molecule were well performed, and 100 clones each were in turn selected from K chain genes and λ chain genes having a higher reactivity. Both were mixed, thereby a library collecting only the clones whose expression and association are well done was obtained and named as KL200.

### 3. Preparation of combination library (Fab antibody gene library) of light chain gene library and heavy chain gene library

### 3-1-1 Isolation of immunogloblin heavy chain gene using PCR

cDMAs were prepared from umbilical blood, bone marrow liquid, lymphocyte in peripheral blood and tonsil using human µ primer (primer 634 shown in the following) or random hexamer by the procedure similar to 2-1, by making each cDNA as a template, and PCR was carried out using 5' primer for acquiring human antibody heavy chain gene (VH1-VH7) and 3' primer (primer in which equal quantities of 4 species of human JH primers were mixed, primer 697-700 shown in the following or human µ primer (primer 634 shown in the following). In Table, the portions underlined are indicated as SfiI sites. Since HVH2a did not correspond to germ line VH2 family, VH2a-2 was newly designed. Moreover, since hVH4a did not correspond to the whole of VH4 family, hVH4a-2 was newly designed. Since VH5a neither corresponded to germ line VH5 subfamily, VH5a-2 was newly designed. Moreover, as a primer corresponding to VH7, hVH7 was designed. These genes were also amplified, then incorporated into pscFvcA-E8VHd, and what kinds of genes were obtained were confirmed by a base sequence. As for hVH5a-2, its sequence is extremely similar to hVH1a, since a gene product similar to a gene product amplified by hVH1a was expected to obtain, it was not used. The PCR product was suspended in 10µL of TE buffer by precipitating with ethanol following phenol treatment. 
634 hum μCH1R (SEQ ID NO.: 46)
ATGGAGTCGGGAAGGAAGTC
 Primers used for the amplification of each VH family Human VH primer SfiI sites are indicated by underlines. 
628 hVH1a (SEQ ID NO.: 47)

GTCCTCGCAACTGCGGCCCAGCCGGCCATGGCC CAGGTGCAGCTGGTGCAGTCTGG
 
629 hVH2a (SEQ ID NO.: 48)

GTCCTCGCAACTGCGGCCCAGCCGGCCATGGCC CAGGTCAACTTAAGGGAGTCTGG
 
630 hVH3a (SEQ ID NO.: 49)

GTCCTCGCAACTGCGGCCCAGCCGGCCATGGCC GAGGTGCAGCTGGTGGAGTCTGG
 
631 hVH4a (SEQ ID NO.: 50)

GTCCTCGCAACTGCGGCCCAGCCGGCCATGGCC CAGGTGCAGCTGCAGGAGTCGGG
 
632 hVH5a (SEQ ID NO.: 51)

GTCCTCGCAACTGCGGCCCAGCCGGCCATGGCC CAGGTGCAGCTGTTGCAGTCTGC
 
633 hVH6a (SEQ ID NO.: 52)

GTCCTCGCAACTGCGGCCCAGCCGGCCATGGCC CAGGTACAGCTGCAGCAGTCAGG
 
629-2 hVH2a-2 (SEQ ID NO.: 53)
GTCCTCGCAACTGCGGCCCAGCCGGCCATGGCC
CAGRTCACCTTGAAGGAGTCTGGTCC
 
631-2 hVH4a-2 (SEQ ID NO.: 54)

GTCCTCGCAACTGCGGCCCAGCCGGCCATGGCC CAGGTGCAGCTACAGCAGTGGGG
 
632-2 hVH5a-2 (SEQ ID NO.: 55)

GTCCTCGCAACTGCGGCCCAGCCGGCCATGGCC GAGGTGCAGCTGGTGCAGTCTGG
 
712 hVH7 (SEQ ID NO.: 56)

GTCCTCGCAACTGCGGCCCAGCCGGCCATGGCC

CAGGTGCAGCTGGTGCAATCTGGGTCTGAGT
 Human JH primer BstPI, XhoI site is shown by an underline.

697 hJH1-2 (SEQ ID NO.: 57)

GGTGGAGGCACTCGAGACGGTGACCAGGGTGC

698 hJH3 (SEQ ID NO.: 58)

GGTGGAGGCACTCGAGACGGTGACCATTGTCC

699 hJH4-5 (SEQ ID NO.: 59)

GGTGGAGGCACTCGAGACGGTGACCAGGGTTC

700 hJH6 (SEQ ID NO.: 60)

GGTGGAGGCACTCGAGACGGTGACCGTGGTCC

| | |
|---|---|
| cDNA | 2µL |
| 10 × buffer #1(Attached to KOD) | 10µL |
| dNTP mixture (2.0 mM) | 10µL |
| 25 m MgC₁₂ | 4µL |
| 5'end side primer (100 pmol/µL) | 1µL |
| 3'end side primer (100 pmol/µL) | 1µL |
| sterilized MilliQ | 71µL |
| KOD DNA polymerase (2.5U/µL; made by Toyobo) | 1µL |

PCR conditions: 35 cycles of PCR carrying out one cycle at 94°C for one minutes, at 55°C for 2 minutes and at 74°C for one minute.

### 3-1-2 Preparation of heavy chain gene library

PCR product obtained in 3-1-1 was treated with restriction enzyme under the following conditions:

| | |
|---|---|
| PCR product | 10µL |
| 10 × K buffer (made by Takara) | 5µL |
| sterilized MilliQ | 33µL |
| HindIII (15 U/µL; made by Takara) | 1µL |
| XhoI (12 U/µL; made by Takara) | 1 µL |

After reacting at 37°C for 2 hours, the portion of 10µL out of it was subjected to agarose electrophoresis, the band nearby 400bp was cut out, and purified by Gene Clean II Kit (made by Funakoshi). The ligation was carried out by reacting it with PCR product treated by restriction enzyme at 16°C for 4 hours to over night under the following conditions:

| | |
|---|---|
| PscFvCA-E8VHd treated with restriction enzyme | 2µL |
| PCR product treated with restriction enzyme | 1µL |
| 10 × ligation buffer | 1.5µL |
| (Attached to T4 DNA ligase) | |
| 10 mM ATP | 1.5 µL |
| sterilized MilliQ | 8µL |
| T4 DNA ligase (10 U/µL; made by Takara) | 1 µL |

### 3-1-3 Introduction of phagemid into E. coli

The obtained DNA was transformed into E. coli DH12S. Concretely, the DNA was once precipitated with ethanol, and dissolved in 3µL of 1/5TE (TE diluted 5-fold with already sterilized MilliQ). Out of this, 1.5µL was suspended in 20µL of competent cell DH12S (made by GIBCO BRL), and transformation was carried out by electroporation method.

### Electroporator

Cell-Porator (Cat. series 1600) made by BRL

| | |
|---|---|
| Setting conditions: voltage booster | 4 kΩ |
| Capacitance | 330µF |
| DC volts | Low Ω |
| Charge rate | Fast |

The above-described transformed E. coli was put in 2 mL of the medium for transformation (SOB), after shaken and cultured at 37°C for one hour, one portion of it was plated on the agar medium (Amp plate), the remains were cultured in 1% glucose, 100µg/mL ampicillin containing 2 × YT medium and stocked with glycerin. The agar medium was incubated at 30°C. the grown colony was separated by picking at it with a stick, a plasmid was prepared, respectively, and the base sequence of the heavy chain gene was examined. These operations were carried out with respect to all of VH1-VH7 and whether or not the clone of interest was obtained was confirmed. The clones of the respective groups (families) were mixed so as to become nearby ratio of the usage frequency in vivo and made it as VH library. The constitutive ratio of the respective families in VH library is indicated as follows:

| family | Usage frequency in vivo (%)* | Constitutive ratio in VL library (%) |
|---|---|---|
| VH1 | 25 | 29** |
| VH2 | 6.6 | 7 |
| VH3 | 40 | 40 |
| VH4 | 19 | 19*** |
| VH5 | 5 | - ** |
| VH6 | 3.8 | 4 |
| VH7 | 1.2 | 2 |

| | | |
|---|---|---|
| * Griffith AD et al. EMBO J. (1994) 13, 3245-60. | | |
| **Actually, since VH1 and VH5 are amplified with the same primers, these cannot be separated and totaled. | | |
| *** cDNA prepared with primer VH4 and cDNA prepared with primer VH4-2 were mixed and made this ratio. | | |

### 3-2 Preparation of combination gene library

200µg of VH library was digested with HindIII and XhoI under the following conditions, the heavy chain gene was cut out and purified by Gene Clean II Kit.

| | |
|---|---|
| 200µg of VH library | 100µL |
| 10 × K buffer (made by Takara) | 40µL |
| sterilized MilliQ | 205µL |
| HindIII (40 U/µL; made by Takara) | 30µL |
| XhoI (50 U/µL; made by Takara) | 25 µL |

The light chain gene clone KL200 confirmed that no deletion exist, and the vector pFCAH9-E8d into which VL library was inserted were also digested with HindIII and XhoI, and the fragment containing the light chain gene was purified by Gene Clean II Kit.

| | |
|---|---|
| pFCAH9-E8d into which KL200 or VL library was inserted 100µg | 100µL |
| 10 × K buffer (made by Takara) | 40µL |
| sterilized MilliQ | 230µL |
| HindIII (40 U/µL; made by Takara) | 15µL |
| XhoI (50 U/µL; made by Takara) | 15 µL |

Next, the ligation was carried out by reacting VH gene library fragment and pFCAH9-E8d vector into which the light chain gene was inserted at 16°C over night under the following conditions:

| | |
|---|---|
| VH library fragment treated with restriction enzyme 10µg | 50µL |
| pFCAH9-E8d containing KL200 or VL library fragments treated with restriction enzyme 40µg | 50µL |
| 10 × ligation buffer | 100µL |
| (Attached to T4 DNA ligase) | |
| 10 mM ATP | 100µL |
| sterilized MilliQ | 670µL |
| T4 DNA ligase (10 U/µL; made by Takara) | 30µL |

E. coli DH12S was transformed using the DNA whose reaction was terminated. Concretely, the DNA was once precipitated with ethanol, and dissolved in 30µL of 1/5TE (TE diluted 5-fold with already sterilized MilliQ). This was suspended in 500µL of competent cell DH12S (made by GIBCO BRL), the electroporation was carried out.

### Electroporator

Cell-Porator (Cat. series 1600) made by BRL

| | |
|---|---|
| Setting conditions: voltage booster | 4 kΩ |
| Capacitance | 330µF |
| DC volts | Low Ω |
| Charge rate | Fast |

The E. coli that the above-described operation were terminated was put in 12 mL of the medium for transformation (SOB), after shaken and cultured at 37°C for one hour, one portion of it was plated on the agar medium (Amp plate), the remains were cultured in 500 mL of 1% glucose, 100µg/mL ampicillin containing 2 × YT medium and stocked with glycerin. The agar medium was incubated at 30°C, and the number of clones was expected from the number of the grown colonies. 4.5 × 10¹⁰ clones were obtained, respectively.

Each VH family which was obtained based on cDNAs synthesized from tonsil mRNA with randam hexamer was cloned into pscFvCA-E8VHd vector, and library combined with KL200 was made as AIMS1 (1.28 × 10¹⁰ independent clones).

Each VH family which was obtained based on cDNA synthesized from umbilical blood, bone marrow liquid, peripheral blood and tonsil mRNA with human m primer was cloned into pscFvCA-E8VHd vector, and gene library combined with KL200 was made as AIMS2 (3.20 × 10¹⁰ independent clones).

Each VH family was obtained based on cDNA synthesized from umbilical blood, bone marrow liquid, peripheral blood and tonsil mRNA with human µ primer, and Each VH family which was combined with VL library was made as AIMS3 (4.50 × 10¹⁰ independent clones).

Furthermore, the above-described combination libraries were mixed at the ratio of (AIMS1+AIMS2): AIMS3 = 1: 1, and made it library composed of 1 × 10¹¹ independent clones (referred to as AIMS4).

### 4. Preparation of scFv-CL antibody gene library

### 4-1-1 Preparation of scNcopFCAH9-E8VHdVLd

3µg (3µL) of pFCAH9-E8d (see reference number 4 of Fig.1) was mixed with 3µL of BstPI (3U/µL), 5µL of 10 × H buffer, 39µL of DW, and treated with restriction enzyme at 37°C for 2 hours. After the treatment, the precipitation obtained by carrying out the precipitation with ethanol was dissolved in 10µL of TE buffer. 1µL of SacI (10U/µL). 5µLof 10 × L buffer, 34µL of DW were mixed into this, and after treated with restriction enzyme at 37°C for 2 hours, agarose gel electrophoresis was carried out, then 4.7kb fragment was collected. The recovered matter was precipitated with ethanol and made it into 10µL (pFCAH9-E8d BstPI-SacI fragment).

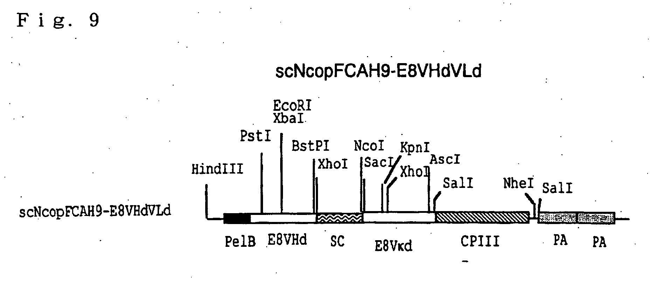

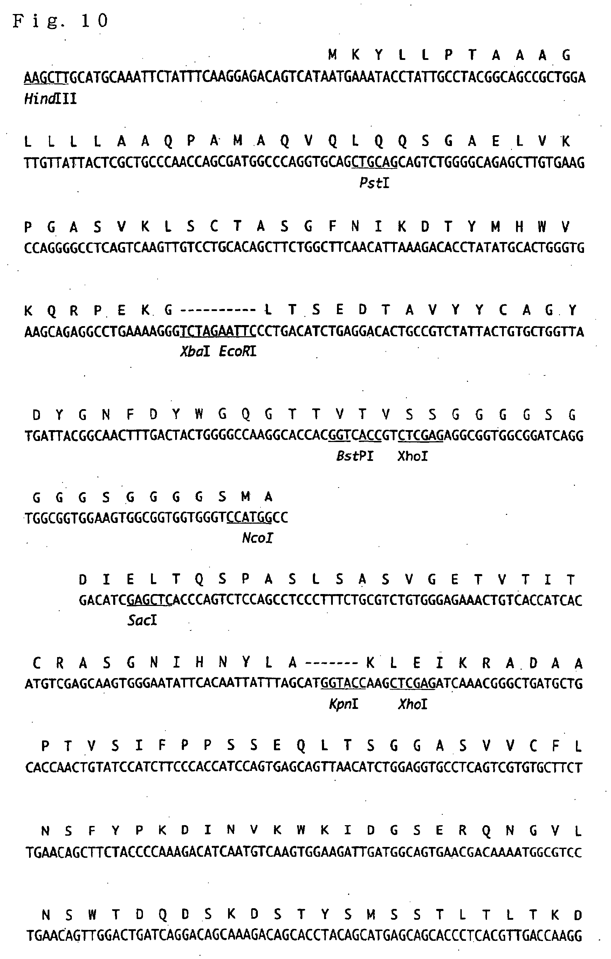

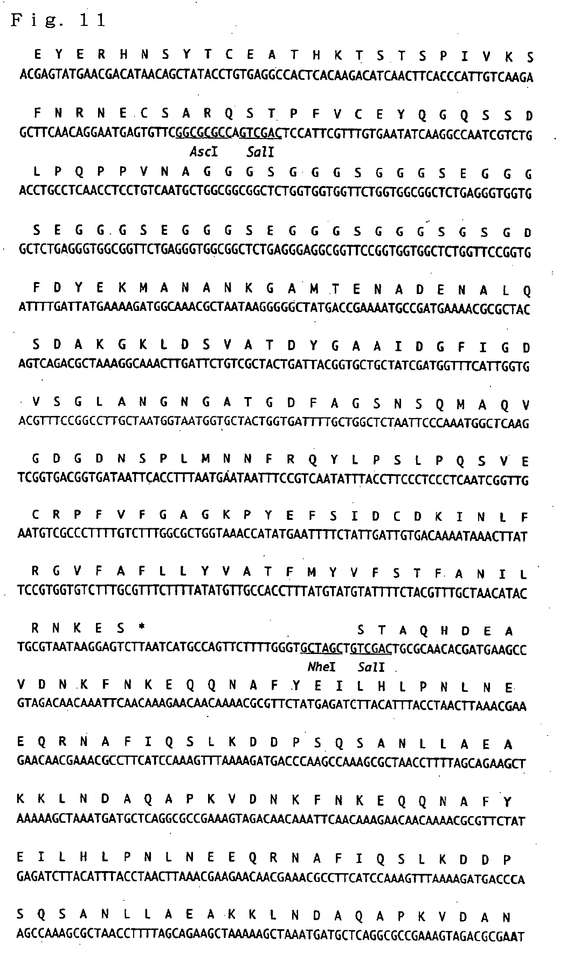

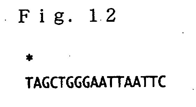

On the other hand, 5µL of primer linF (100pmol/µL) and 5µL of primer linR(100pmol/µL) were mixed, and after heated at 94°C for 5 minutes, and annealed by being heated at 80°C for 5 minutes, at 70°C for 5 minutes and left at room temperature for 30 minutes. 2µL was taken, then 1µL of pFCAH9-E8d BstPI-SacI fragment obtained above, 1.5µL of 10 × ligation buffer, 9.5µL of DW and 1µL of T4DNA ligase were mixed, and reacted at 16°C for 16 hours. After the reaction the precipitation was carried out with ethanol resulting in being condensed into 3µL, and out of this, using 1.5µL, 20µL of E. coli DH12S competent cell was transformed by electroporation. The plasmid of the obtained clone was extracted, the base sequence was confirmed and named as scNcopFCAH9-E8VHdVLd. In Fig.9, the structure of scNcopFCAH9-E8VHdVLd is schematically shown. Moreover, in Fig.10-Fig.12, the base sequence of the insert portion of scNcopFCAH9-E8VHdVLd and amino acid sequence coded by it are shown.

Primer linF (SEQ ID No.: 61)

GTCACCGTCTCGAGAGGCGGTGGCGGATCAGGTGGCGGTGGAAGTGGCGGTGGTGGGTC

CATGGCCGACATCGAGCT

Primer linR (SEQ ID No.: 62)

CGATGTCGGCCATGGACCCACCACCGCCACTTCCACCGCCACCTGATCCGCCACCGCCT

CTCGAGACG

### 4-1-2 Incorporation of AIMS-4L chain into scNcopFCAH9-E8VHdVLd

20µg (20µL) of scNcopFCAH9-E8VHdVLd was mixed with 30µL of 10 × NEB4 buffer, 30µL of 10 × BSA, 10µL of NcoI (10U/µL) and 10µL of AscI (10U/µL), and treated with restriction enzyme at 37°C for 2 hours. 4.5kb fragment was recovered by agarose gel electrophoresis, precipitated with ethanol and made it into 10µL (5µL of scNcopFCAH9-E8VHdVLd NcoI-AscI fragment was obtained). On the other hand, 20µg (20µL) of AIMS-4 library plasmid (plasmid constituting AIMS-4 library) was mixed with 30µL of 10 × NEB4 buffer, 30µL of 10 × BSA, 10µL of NcoI (10U/µL) and 10µL of AscI (16U/µL), and reacted at 37°C for 2 hours. The agarose gel electrophoresis was carried out, 680bp fragment was recovered, precipitated with ethanol and made it into 10µL (1µg of AIMS-4 library plasmid NcoI-AscI fragment was obtained).

scNcopFCAH9-E8VHdVLd NcoI-AscI fragment 2. 5µg(5µL), AIMS-4 library plasmid NcoI-AscI fragment 1µg (10µL), 10 × ligation buffer (10µL) , DW (72µL) and T4 DNA ligase (3µL) were mixed and reacted at 16°C for 16 hours. It was precipitated with ethanol, condensed into 3µL, out of this, using 1.5µL, 0.2 mL of E. coli DH12S competent cell was transformed by electroporation. Following the electroporation, the DH12S was put in the SOC medium, after cultured at 37°C for one hour, and cultured at 30°C over night in 2L of TYGA medium. One portion was plated on the agar medium, and when the total number of clones were estimated, it was 2.1 × 10⁸. When plasmid was extracted from E. coli cultured over night, 2mg was obtained. This was made AIMS-4 VL library plasmid.

### Incorporation of AIMS-4H chain into AIMS-4 VL library plasmid and transformation

500µg(200µL) of AIMS-4 VL library plasmid obtained in 4-1-2 was mixed with 20µL of HindIII(50U/µL), 40µL of 10 × M buffer and 240µL of DW, and after it was treated with restriction enzyme at 37°C for 2 hours, 10µL of BAP C75 (0.4U/µL) was added, and reacted at 37°C for 2 hours, at 50°C for 15 minutes. After phenol treatment, precipitated with ethanol, and dissolved into 20µL of TE buffer. 20µL of XhoI (50U/µL), 40µL of 10 × H buffer and 320µL of DW were-mixed with this, and treated with restriction enzyme at 37°C for 2 hours. This was subjected to agarose gel electrophoresis, 4.5kb fragment was recovered , precipitated with ethanol and made it into 40µL (220µg of AIMS-4 VL library plasmid XhoI-HindIII BAP fragment was obtained).

On the other hand, 600µg(600µL) of AIMS-4 library plasmid was mixed with 20µL of HindIII(50U/µL), 80µL of 10 × M buffer and 100µL of DW, and after it was cut at 37°C for 2 hours. After phenol treatment, precipitated with ethanol, and dissolved into 50µL of TE buffer. 20µL of XhoI (50U/µL), 40µL of 10 × H buffer and 290µL of Dw were mixed with this, and treated with restriction enzyme at 37°C for 2 hours. Then, 10µL of BAP C75 (0.4U/µL) was added, reacted at 37°C for 2 hours, at 50°C for 15 minutes. This was subjected to agarose gel electrophoresis, 0.5kb fragment was recovered , precipitated with ethanol and made it into 40µL (41µg of AIMS-4 VL library plasmid XhoI BAP-HindIII BAP fragment was obtained).

50µL (9.1µL) of AIMS-4 VL library plasmid XhoI-HindIII BAP fragment, 41µL (40µL) of AIMS-4 library plasmid XhoI BAP-HindIII BAP fragment, 30µL of 10 × ligation buffer, 195.9µL of DW and 25µL of T4 DNA ligase were mixed and reacted at 16°C for 16 hours. This was subjected to agarose gel electrophoresis, 4.5kb fragment was recovered , precipitated with ethanol and made it into 36µL (35µg was obtained) with 1/10TE buffer. 5µL of 10 × kination buffer, 5µL of 10mM ATP and 4µL(10U/µL) of T4 polynucleotide kinase were added to this and reacted at 37°C for one hour. Furthermore, 1000µL of 10 × ligation buffer and 8750µL of DW and 200µL of T4 DNA ligase were mixed, reacted at 16°C for 16 hours, self-ligation was carried out. After condensed with 1-butanol, it was precipitated with ethanol, made it into 200µLwith 1/10TE, using this, 5 mL of E. coli DH12S competent cell was transformed by electroporation. Following the electroporation, DH12S was put in 50mL of SOC medium, after cultured at 37°C for 30 minutes, it was cultured at 30°C over night in 2L of TYGA medium. One portion was put in the agar medium, and when the total number of clones were estimated, it was 1.1 × 10¹¹. The library composed of the assembly of clones thus obtained was made as AIMS-5. In Fig. 13 a), the fundamental structure of clone constituting the AIMS-5 antibody gene library is shown.

### 4-2 Preparation of scFv-CL antibody phage library from scFv-CL antibody gene library (AIMS-5)

2.5mL each of AIMS-5 suspension was added to 16 bottles of flasks of 5 L into which 300mL of 2 × YT medium has been inputted and then 100µg/mL ampicillin was added, then it was shaken and cultured at 37°C and grown until the absorbance became 1.0 while the absorbance at 600nm of wavelength was measured every hour. 12mL per one bottle of flask of helper phage liquid (M13KO7) was added to the cultured liquid, infected with the helper phage, cultured at 37°C for 2 hours, and made it DH12S already infected with helper phage.

600mL of 2 × YT medium, 0.6mL of ampicillin(100µg/mL), 0. 8mL of Kanamycin (50µg/mL) and 200mL of DH12S already infected with helper phage were added to 24 bottles of flasks of 5L and shaken and cultured at 37C° for 20 hours.

The bacterial cells were centrifuged at 8000 rpm at 4°C for 10 minutes, and supernatant was recovered . 4L of 20% polyethylene glycol/2 . 5MNaCl was added to the supernatant, after it was quietly stirred for about 20 minutes, centrifuged at 8000rpm at 4°C for 20 minutes. The supernatant was discarded and further, centrifuged at 8000rpm at 4°C for 3 minutes, and the precipitate was recovered . The precipitate was dissolved in PBS to which 0.05% NaN₃ was added, after it was centrifuged at 1000rpm at 4°C for 15 minutes and the supernatant was recovered, further, centrifuged at 8000rpm at 4°C for 3 minutes and the supernatant was recovered.

The titer of the recovered phage solution was checked as followings: the phage solution was diluted with PBS in 10⁶,10⁷ and 10⁸-fold, out of these, 10µL was infected with 990µL of DH12S, cultured at 37°C for one hour. 100µL of this was plated on LBGA plate and cultured at 30°C for 18 hours. The titer of the stock solution before dilution was calculated by counting the number of colonies. The stock solution of the phage solution was suspended in PBS containing 2% skimmed milk and 0.05% NaN₃ so as to be 2 × 10¹⁴/mL.

### 5. Selection of phage capable of specifically binding to specific antigen from scFv-CL antibody library

In the present Example, as a model antibody, the expression product in "CC046" E. coli which is a cDNA considered as expressed within the cell of C. elegans was used. Concretely, the portion (CC046 antigen) corresponding to 96 amino acid of N-terminal region of CC046 was used. The selection (screening) of phage specifically binding to CC046 antigen was carried out by panning method.

### 5-1 Preparation of test tube for screening

CC046 antigen was prepared into 20µg/mL, with PBS, 3mL each of this was added to 3 test tubes (Maxisorp; made by Nunc), incubated at 4°C for 18 hours, and the antigen were absorbed on the inner surface of the test tubes. After the absorption, the antigen solutions were discarded, 3mL each of 2% skimmed milk containing PBS solution was added, reacted at 25°C for one hour, and the blocking was carried out for preventing the phage antibody from nonspecifically binding to the test tube.

### 5-2 Screening operation

AIMS-5 library obtained in 4 was dissolved so as to be 1 × 10¹⁴ CFU/9mL in PBS containing 2% skimmed milk and 0.1% Tween 20. 3 mL each of the solution thus prepared was added to the 3 test tubes(the antigen already absorbed test tubes) prepared in the above operation, then after reaction at 25°C for 2 hours, these tubes was washed 4 times with PBS to which 0.1% Tween 20 has been added, washed 4 times with PBS, and washed once with sterilized extrapure water(prepared with MilliQ).

Subsequently, the phage bound to the antigen binding test tube was recovered as follows: 3mL per a test tube of 0. 1M triethyl amine (pH 12.3) was added, after it was reacted at room temperature for 20 minutes using a rotator and dissociated, 1.1mL of 1M Tris-HCl buffer (pH 6.8) was added and neutralized, and this liquid was recovered.

### 5-3 Amplification of recovered phage

The recovered liquid was treated with (infection of phage withE. coli), (infection of helper phage) and (recovery of phage), and the contained phage was purified and amplified.

### 1) Infection of phage with E. coli

E. coli (DH12S) was cultured in 50mL of 2 × YT medium, grown so that the absorbance at the wavelength of 600nm became 0.5, the phage liquid dissociated in the described above was added and shaken and cultured at 37°C for one hour.

### 2) Infection of helper phage

1) 62.3mL of culture solution was taken, 425mL of 2 YT medium, 12.5mL of 40% glucose and 0. 5mL of ampicillin (100µg/mL) were added, and after it was cultured at 37°C until the absorbance at the wavelength of 600nm became 0.5, the bacterial cells were precipitated by centrifuging at 5000 rpm at 4°C for 10 minutes, recovered and suspended in 300mL of 2 × YT medium to which 0.3mL of ampicillin (100mg/mL) was added. 1/100 of helper phage M13KO7 (3 × 10¹⁰cfu/mL) was added to this and it was shaken and cultured at 37°C for one hour.

The culture solution was added to 900mL of the medium previously warmed to 37°C (solution that ampicillin (100µg/mL) and Kanamycin (70µg/mL) were added to 2 × YT medium), and cultured at 37°C over night.

### 3) Recovery of phage

The culture solution of 2) was centrifuged at 7000rpm at 4°C for 10 minutes, 1/5 volume of 20% polyethylene glycol to which 2.5M sodium chloride had been added to the supernatant, and after it was quietly stood at room temperature for 20 minutes, it was centrifuged at 8000rpm at 4°C for 15 minutes and the precipitated matter was recovered, sterilized PBS of 1/10 of the culture solution was added and dissolved, 1/5 of 20% polyethylene glycol to which again 2. 5M sodium chloride has been added was added, centrifuged at 10000rpm at 4°C for 20 minutes, the supernatant was discarded, and further, spinned down, that is, centrifuged at 10000rpm at 4°C for 2 minutes. The PBS to which 0.05% NaN₃ has been added was added to 1/100 of the culture solution, and the precipitate was dissolved and the phage was recovered.

### 5-4 Re-screening with amplified phage

The screening was repeated using the antigen binding test tube similar to 5-2 by utilizing the amplified phage. Since the washing in the screening is an important step when the phage specifically absorbed was dissociated and the phage having a high binding avidity is selected, the washing conditions of the screening on and after the second time were made as follows:
Second time: Washing 6 times with PBS+0.1% Tween 20, washing 6 times with PBS, and washing once with sterilized extrapure water
Third time: Washing 13 times with PBS+0.1% Tween 20, washing 13 times with PBS, and washing once with sterilized extrapure water

### 5-5 Measurement of antigen binding activity (affinity) of antibody obtained by screening

The present inventors have found that the phage obtained by the screening described above is made infected with E. coli, and subsequently is cultured for a long time without being super infected with helperphages, whereby cp3 fusion type scFv-CL antibody is secreted out of E. coli.

On the basis of the above fact, the measurement of antigen biding activity of a scFv-CL antibody coded by phage was carried out not using the antibody expressed on the surface of the phage but using the cp3 fusion type scFv-CL antibody. A concrete method is as follows:

### 5-5-1 Expression derivation of cp3 fusion type scFv-CL antibody

First, E. coil DH12S was infected with the phage obtained by the screening described above. Next, E. coli which was infected with the phage was plated on an agar medium containing a YTGA (1% glucose, 100µg/mL ampicillin containing 2 × YT medium), several tens of colonies were selected from the grown colonies, clones were inputted in 3mL of 2 × YT medium into which 1% glucose and 100µg/mL ampicillin have been added, respectively and cultured over night. 100µL of the culture solution cultured over night was inputted in 10mL of 2 × YT medium into which 0.1% glucose and 100µg/mL ampicillin have been added, when it has reached logarithmic growth phase, 10µL of 1M IPTG (isopropyl-1-thio-β-D-galactoside) was added, and further, after it was cultured at 30°C for 21 hours, 1.5 mL of the culture solution was taken in a Eppendorg tube, centrifuged at 10000rpm at 4°C for 5 minutes, then its culture supernatant was taken, sodium azide was added so as to be 0.1%, and made it as a sample.

### 5-5-2 Measurement of antigen binding activity (affinity) by ELISA method

First, plates for ELISA were prepared as follows: 100µL of CC046 antigen (10µg/mL) was added to each well of 96 well microtiter plate (Maxisorp; made by Nunc), made bound at 4°C for 18 hours, 200µL of 5%BSA containing phosphate buffer (blocking solution) was added to each well, and blocked at 37°C for one hour. After the blocking solution was discarded, washed with PBS once, and used for measurement of the affinity.

100µL each of the sample obtained in 5-5-1 was added to each well of the plate obtained in this way, and reacted at 25°C for one hour. After the reaction, it was washed 4 times with PBS, 100µL of 500-fold diluted rabbit anti-cp3 antibody (made by MBL) was added and reacted at 25°C for one hour. After each well was washed with PBS 4 times, 100µL of 1000-fold diluted HRP labeled goat anti-rabbit IgG antibody (made by MBL) was added. and reacted at 25°C for one hour. Again, it was washed 4 times with PBS, 100µL of solution of orthophenylene diamine and hydrogen peroxide was added, and after it was reacted for a while, 100µL of 1.5N phosphate was added and the reaction was stopped, and the absorbance at the wavelength of 492nm was measured. As a result, it has been confirmed that 21 clones out of 25 clones have the activity.

When the DNA base sequence determination of the base sequence was carried out on these 21 clones, it is understood that these are 7 kinds of clones. The analysis of DNA base sequence was carried out using a DNA sequencer (made by LI-COR) by the known method (dideoxy method).

### 5-5-3 Comparison of antigen binding activity of each clone by immunofluorescence staining method

The antigen binding activities of 7 kinds of the clones obtained in 5 - 5 - 2 were compared by an immunofluorescence staining method. For material of immunofluorescence, the early embryo of C. elegans was used.

First, C. elegans was fed using a well known method, and the fertilized ovum was obtained. After the fertilized ovum was grown to the early embryo, taken on the slide glass, the whole slide glass was immersed in ethanol cooled to -20°C for 10 minutes. Next, it was immersed in acetone cooled to -20°C for 10 minutes. Left at room temperature, when the acetone was volatilized, it was immersed in PBS for 5 minutes. Subsequently, it was immersed in PBS containing 5% skimmed milk for blocking.

Next, the culture supernatant of the sample (scFv-CL antibody solution) which was positive (the activity was recognized) in 5-5-2 was diluted 2-fold with PBS containing 5% skimmed milk, 25µL of this was added to the early embryo on the slide glass prepared in the described above, and reacted at room temperature for 2 hours.

After the reaction, the antibody solution was removed from the slide glass, the washing was carried out by immersing in the washing liquid (0.1M Tris-HCl pH7.5, 0.2M NaCl, 0.1% Tween) for 10 minutes. The washing liquid was replaced and further 2 times the same operation was repeated.

Next, 25µL of rabbit anti-cp3 antibody (prepared by immunizing the rabbit with cp3 protein) which has been diluted 200-fold with PBS containing 5% skimmed milk was added to the early embryo on the slide glass, and reacted at room temperature for 2 hours. When the reaction was terminated, the antibody liquid was removed from the slide glass, the washing was carried out by immersing in the washing liquid at room temperature for 10 minutes. The washing liquid was replaced, and further 2 times the same operation was repeated.

Subsequently, 25µL of Cy3 labeled anti-rabbit IgG antibody which has been diluted 800-fold in PBS containing 5% skimmed milk was dropped on the slide glass, and reacted at 4°C over night.

When the reaction was terminated, the antibody solution was removed from the slide glass, it was washed by immersing in the washing liquid (0.1MTris-HC1 pH7.5. 0.2MNaCl. 0 .1% Tween at room temperature for 10 minutes. The washing liquid was replaced and the same operation was repeated once again.

Subsequently, 1µL of 1mg/mL DAPI was added to 50mL of the washing liquid and the slide glass was immersed in this at room temperature for 10 minutes.

After the liquid was removed, mounting agent was mounted, covered with the cover glass and the microscope observation was carried out.

### 5-5-4 Comparison of antigen binding activity of each clone by Western blotting method

The antigen binding activities of the respective clones obtained in 5-5-2 were compared by the well known method shown in the following.

The portion of 100ng or 10 ng per one lane of CC046 purifed antigen was subjected to the electrophoresis by SDS-PAGE. The SDS-PAGE gel was transferred to the membrane.

After the membrane was blocked, the culture supernatant of each clone obtained in 5-5-2 was diluted 4-fold (under the coexistence of 5% skimmed milk), mounted on the membrane and reacted.

It was washed 3 times with 0.05% Tween 20-PBS after the reaction at room temperature for one hour. The anti-cp3 antibody (500-fold diluted, under the coexistence of 5% skimmed milk) was added to the membrane already washed, and reacted at room temperature for one hour. After the reaction, it was washed 3 times with 0.05% Tween 20-PBS.

HRP labeled goat anti-rabbit IgG antibody (× 1000, MBL code No.458) was added to the membrane already washed, and reacted at room temperature for one hour. After the reaction, it was washed 3 times with 0.05% Tween 20-PBS.

The membrane was immersed in a chemical light emitting reagent (Renaissance; Western Blot Chemiluminescence Reagent Plus, NEN Life Science Products, Inc.), was wrapped with a lap, and the film was exposed to the light. The film was developed and the results were evaluated.

From the results described above, and the results of 5-5-3, the clone having the highest antigen binding activity was selected (referred to as clone CC046N2), and used in the experiments hereinafter.

### 6. Conversion from cp3 fusion type scFv-CL antibody to GFP fusion type scFv-CL antibody

### 6-1 Preparation of vector for expressing GFP fusion type scFv-CL antibody

As shown in Fig.13 b) lower portion, the vector for expression pAscHGFP was prepared by adding AscI site between the start codon and His-tag sequence of p6 × His-GFP vector (made by Clontech).

By inserting a scFv-CL gene at AscI site of this vector, a vector for expressing an actual antibody protein will be completed (Fig.13 c)). The antibody expressed by this vector is a scFv-CL type, and GFP is to be connected to the CL region via His-tag.

The concrete operation that adds AscI site was carried out as the following: 0.5µL of p6 × His-GFP vector (0.1mg/mL), 10µL of 10 × LA PCR buffer II, 16µL of dNTP mixture, 10µL of 25mM MgCl₂, 1µL of primer GFPAscF (100pmol/µL), 1µL of primer GFPAscR (100pmol/µL), 1µL of LA Taq polymerase (5U/µL) and 60.5µL of sterilized water were mixed on ice, two drops of mineral oil were added, and the temperature was maintained at 94°C for 5 minutes. The incubation cycle composed of a reaction at 94°C for one minute, a reaction at 55°C for one minutes and a reaction at 72°C for 4 minutes were repeated 30 times and further, it was incubated at 72°C for 7 minutes.

GFPAscFprimer (SEQ ID NO.: 63)

TAGGCGCGCCAGGGGGGTTCTCATCAT (27mer)

GFPAscRprimer (SEQ ID NO.: 64)

AAGGCGCGCCATGGTTTATTCCTCCTT (27mer)

After the obtained PCR product was confirmed by agarose gel electrophoresis, it was treated with phenol, precipitated with ethanol, and condensed into 10µL. 10µL of 10 × NEB4 buffer, 75µL of sterilized water and 5µL of AscI (10U/µL) were added to this, and the temperature was maintained at 37°C for 3 hours. The fragment of interest was recovered by carrying out the agarose electrophoresis of 1/5 of the volume, precipitated with ethanol, condensed, and dissolved in 30µL of TE buffer. By not subjecting this to the ligation reaction, but inducing a self-ligation by a well known method, a cyclic plasmid vector can be constructed.

According to the method similar to the described above, vector pAscHRFP for expressing RFP (Red Fluorescent Protein) fusion type scFv-CL antibody can be prepared. Moreover, if the following operations is carried out using this, RFP fusion type scFv-CL antibody can be expressed.

### 6-2 Amplification of scFv-CL antibody gene from clone CC046N2

A scFv-CL antibody gene was amplified using the clone CC046N2 by a PCR method. At this time, two primers containing AscI site were synthesized, using these, SfiI site of N-terminal of VH gene was converted into AscI site under the conditions where amino acid sequence coded by scFv-CL antibody gene portion is not changed. The scFv-CL antibody gene amplified is schematically shown in the upper stage of Fig.13 b).

The primer using PCR method will be shown in the following. It should be noted that the small letter portion is AscI site.

Forward primer (Asc3F) (SEQ ID NO.: 65)
5 -AAggcgcgccAGGTGCAGCTGGTGGAGTCTG-3

Reverse primer (AscR) (SEQ ID NO.: 66)
5'-GTCGACTggcgcgcc-3

The concrete method of PCR method will be indicated in the following:

0.5µL of clone CC046N2, 10µL of 10 × buffer #1, 10µL of dNTP mixture, 4µL of 25mMMgCl₂, 1µL of primer Asc3F (100pmol/µL), 1µL of primer AscR (100pmol/µL) , 1µL of KOD polymerase (2.5U/µL) and 72.5µL of sterilized water were mixed on ice, two drops of mineral oil were added, and the temperature was maintained at 94°C for one minutes. One incubation carried out cycle at 94°C for one minutes, 55°C for one minutes and at 72°C for one minute was repeated until 25 cycles. After the obtained PCR product was confirmed by agarose gel electrophoresis, it was treated with phenol, precipitated with ethanol, and condensed into 10µL.

After the obtained PCR product was confirmed by agarose gel electrophoresis, after it was treated with phenol, it was precipitated with ethanol, and suspended in 10µL of TE buffer.

### 6-3 Introduction of scFv-CL antibody gene into expression vector

The PCR product obtained in 6-2 was treated with restriction enzyme under the following conditions:

| | |
|---|---|
| PCR product | 10µL |
| 10 × NEB4 buffer(made by New England Biolabs) | 10µL |
| Already sterilized MilliQ | 75µL |
| AscI (made by New England Biolabs; 10U/µL) | 5µL |

After incubated at 37°C for 3 hours. ½ volume of the solution was subjected to electrophoresis, thereby a fragment of interest was recovered, then condensed by precipitation with ethanol followed by dissolution into 30µL of TE.

On the other hand, similarly, vector for expression pAscHGFP (Fig.3 b) lower portion) was treated with restriction enzyme and purified by Gene Clean II Kit.

Next, the ligation was carried out by reacting the PCR product treated above and a vector for expression at 16°C for 4 hours over night under the following conditions:

| | |
|---|---|
| pAscHGFP treated with restriction enzyme | 2µL |
| PCR product treated with restriction enzyme | 1µL |
| 10 × ligation buffer | 1.5µL |
| (Attached to T4 DNA ligase) | |
| 10 mM ATP | 1.5µL |
| sterilized MilliQ | 8µL |
| T4 DNA ligase (10 U/µL; made by Takara) | 1µL |

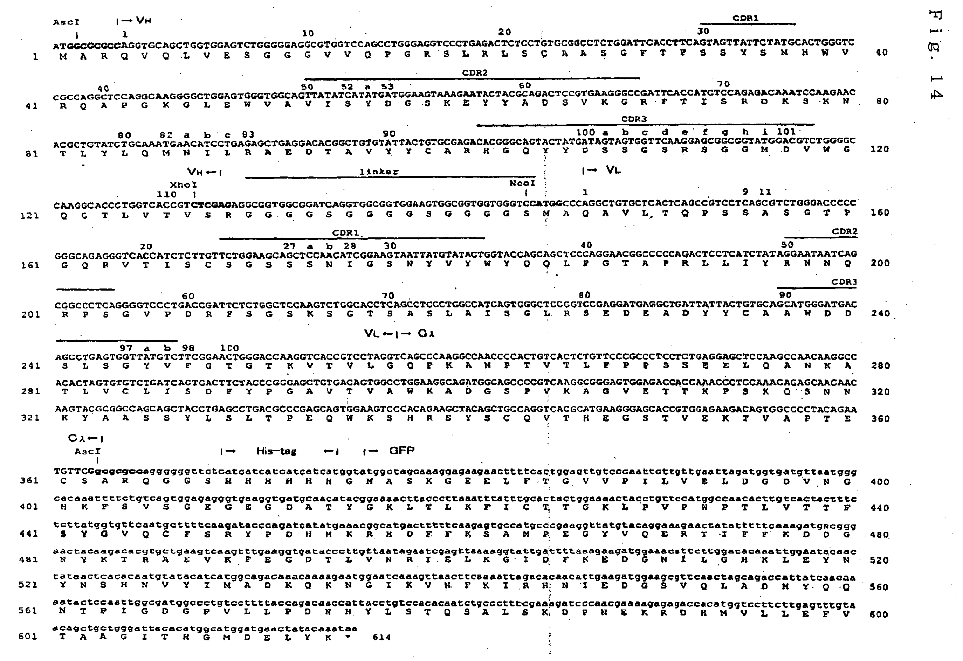

The vector (pscFv(CC046N2)-CL-GFP plasmid) obtained as the results of the above-described operation was utilized for GFP fusion type scFv-CL antibody preparation. The DNA sequence and aminoacid sequence of pscFv(CC046N2)-CL-GFP plasmid are shown in Fig.14.

### 7. Production of GFP fusion type scFv-CL antibody within E. coli

### 7-1 Introduction of pscFv(CC046N2)-CL-GFP plasmid into E. coli

E. coli DH12S was transformed using pscFv(CC046N2)-CL-GFP plasmid by electroporation as follows:

Concretely, pscFv(CC046N2) -CL-GFP plasmid was dissolved in 3µL of 1/10 TE, which was suspended in 20µL of competent cell DH12S (made by GIBCO BRL), the electroporation was carried out under the following conditions:

### Electroporator

Cell-Porator (Cat. series 1600) made by BRL

| | |
|---|---|
| Setting conditions: voltage booster | 4 kΩ |
| Capacitance | 330µF |
| DC volts | Low Ω |
| Charge rate | Fast |

E. coli the above-described operation was terminated was inputted in 2 mL of the medium for transformation (SOB), after shaken and cultured at 37°C for one hour, it was cultured on the TYGA agar medium. As for transformant 24 clones, a plasmid DNA was prepared from E. coil using a well known method, it was cut with the restriction enzyme PvII, and subjected to agarose gel electrophoresis. According to the results (in the case of the correct direction, the fragments having the length of 0.3, 1.7 and 4.3kb are obtained), the clones whose direction of the inserted scFv-CL gene is correct were selected.

### 8. Purification and analysis of GFP fusion type scFv-CL antibody

### 8-1 Induction of expression and purification of GFP fusion type scFv-CL antibody

First, E. coli having pscFv(CC046N2)-CL-GFP plasmid, which was obtained in 7-1, was suspended in 1L of 100µg/mL ampicillin containing 2 × YT medium, and cultured at 30°C. The concentration of E. coil was measured by the absorbance at the wavelength of 600nm, and when it became log phase, IPTG was added so as to be 1mM, and further cultured at 30°C for 21 hours. Next, the bacterial cells were recovered by centrifuging the culture solution, 30mL of 50mM Na-phosphate buffer/300mM NaCI pH8 was added to the bacterial cells, and the bacterial cells were broken by supersonic treatment. The broken liquid was centrifuged and 30mL each of the supernatant was applied to Ni-NTA agarose column (gel capacity 2.8mL) which had been equilibrated. After the column was washed with 250mL of 50mM sodium phosphate/300mM NaCl pH8, elution was performed with 1mL of 10, 20, 30, 40, 50, 60, 70, 80, 90, 100, 120, 150, 200 and 250mM imidazole. The light having the wavelength of 366nm was irradiated at each eluted fraction, and the fractions( 30-90mM) emitting the fluorescence were selected. As a result of measuring the protein concentration of the selected fractions, about 200µg of protein was obtained. The following analysis was performed using this purified protein solution.

### 8-2 Analysis of purified protein by SDS-PAGE

The purified protein solution obtained in 8-1 was analyzed by SDS-PAGE. It should be noted that as Comparative Example, the bacterial cells broken liquid (extracted crude liquid) prior to the purification was used.

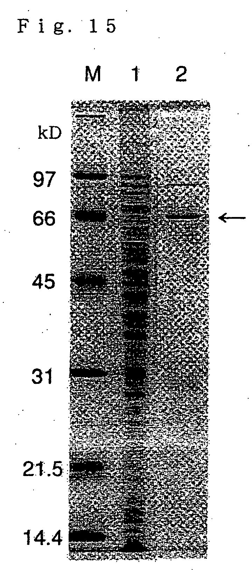

The results of SDS-PAGE are shown in Fig.15. In Fig.15, lane 2 is a lane in which the purified protein solution was flowed, Moreover, in lane M and lane 1, molecular weight marker and the bacterial cells broken liquid prior to the purification which is comparative Example were electrophoresed, respectively.

As shown in Fig. 15, although in the crude extract, it could not determined because of the admixed protein, in the purified protein solution, it was condensed, and the band could be clearly detected at the location that had been expected (location indicated by arrow).

### 8-3 Measurement of antigen binding activity (affinity) by ELISA method of purified protein

The binding activity with respect to the antigen (CC046) of the purified protein (GFP fusion type scFv-CL antibody) obtained in 8-1 was measured by ELISA method. The concrete method and conditions are indicated as follows:

First, the plate for ELISA was prepared as follows: After 100µL of the antigen (20µg/mL) was added to each well of 96 well microtiter plate (Maxisorp; made by Nunc) and bound at 4°C for 18 hours, 200µL of 5% BAS (blocking solution) was added to each well and blocked at 37°C for one hour. After the blocking solution was discarded, it was washed with PBS once, and used for measuring the affinity.

100µL each of maltiplicatively diluted purified solution obtained in 8-1 was added to each well of the plate thus obtained, and reacted at 37°C for one hour. After the reaction, it was washed with PBS 4 times, 100µL of 5000-fold diluted rabbit anti-GFP antibody (made by MBL) was added, and reacted at 37°C for one hour. After each well was washed with PBS 4 times, 100µL of HRP labeled goat anti-rabbit IgG antibody (made by MBL) which has been diluted 10000-fold was added, reacted at 37°C for one hour. After each well was washed 4 times with PBS, 100µL of a coloring substrate solution (OPD) was added and the absorbance at the wavelength of 492nm was measured by a plate reader as described above.

As a result, the antibody activity comparable to cp3 fusion type saFv-CL antibody in 5-5-3 was recognized.

### 8-4 Immunofluorescence staining using purified protein solution

Next, for the purpose that it is proved that in the purified protein solution obtained in 8-1, an antibody molecule specifically binds to the antigen CC046 and emits the fluorescence exists, immunofluorescence stainings were carried out on the embryo cell of C. elegans in the various stages.

First, the purified protein solution obtained in 8-1 (scFv-CL-GFP) was diluted with 5% skimmed milk containing PBS into 1µg/mL, 25µL of it was added to the slide glass to which C. elegans prepared by the gist described above is bound, and reacted at 4°C over night.

Next, the antibody solution was removed from the slide glass, immersed in the washing liquid (0. 1M Tris-HCl pH7 .5, 0. 2M NaCl, 0.1% Tween) at room temperature for 10 minutes, the washing liquid was replaced and once more the same operation was repeated.

1µL of DAPI (1mg/mL) was added to 50mL of washing liquid, and in which the slide glass was immersed at room temperature for 10 minutes.

After the liquid was removed, the mounting agent was mounted, covered with the cover glass, and the microscopic observation was performed.

For the observation of the staining image of the sample prepared in this way, Filter set No.10 (excitation filter BP450-490, dichroic mirror FT510, barrier filter BP515-565) (made by Zeiss) was used. It should be noted that DNA staining with DAPI was carried out at the same time.

The results of observing the early embryo are shown in Fig. 16a. The fluorescence by GFP is observed at the portion that the arrow A indicates (cell center portion). Specifically, the existence of a molecule for clearly recognizing the antigen CC046 is confirmed. Hence, the existence of GFP fusion type scFv-CL antibody specifically recognizing CC046 and emitting the fluorescence have been confirmed in the purified protein solution. Moreover, it is understood that CC046 locally exists at cell center. It should be noted that at the portion indicated by the arrow d, the fluorescence by DAPI is observed.

Moreover, Fig.16b shows the results that a similar immunofluorescence staining was carried out using cp3 fusion type scFv-CL antibody acquired from the clone obtained in 5-5. In this case, as a secondary antibody, as described above, rabbit anti-cp3 antibody, as a tertiary antibody, Cyanin3 labeled goat anti-rabbit IgG antibody (Jackson Immuno Research Laboratoried) were used. The floorescence is observed at the portion indicated by the arrow B. It should be noted that at the portion indicated by the arrow D, the fluorescence by DAPI is observed.

By Comparison with Fig. 16a, the case where GFP fusion type scFv-CL antibody was used is better than the case where cp3 fusion type scFv-CL antibody was used from the viewpoints of sensitivity and resolution. It should be noted that for the observation of the staining image in the case where cp3 fusion type scFv-CL antibody was used, Filter set No.15 (excitation filer BP456/12. dichroic mirror FT580, barrier filter LP590) (made by Zeiss) was used. Moreover, similarly to the case of GFP fusion type, DNA staining was carried out at the same time.

In the present Example, "5. Selection of phage specifically binding to specific antigen from scFv-CL antibody library" could be done for about 2 weeks. Moreover, the procedures from "6. Conversion from cp3 fusion type scFv-CL antibody to GFP fusion type scFv-CL antibody to "7. Production of GFP fusion type scFv-CL antibody within E. coli", could be done for about one week. Therefore, it means that it is not required for one month to isolate GFP fusion type scFv-CL antibody which is the final form. In this way, it is possible that scFV type antibody with which fluorescent protein has been fused is acquired in an extremely short period according to a method of the present invention. It should be noted that the period required for the preparation of scFv type antibody with which fluorescent protein has been fused is capable of being further shortened-according to the experimental environment.

The present invention is not limited by the descriptions of Embodiments and Examples of the above-described invention at all. The present invention also includes a variety of modified aspects in the scope where those skilled in the art can easily conceive without departing the scope of the claims.

### INDUSTRIAL APPLICABILITY

According to the present invention, a novel method for preparing a scFv type antibody with which a fluorescent protein has been fused is provided. According to a method for preparation of the present invention, the desired antibody can be obtained in an extreme short period. A variety of antibodies, each of which recognizes its antigen are obtained by utilizing a scFv antibody library having the number of clones enough to contain the intravital diversity.

Moreover, since a scFv type antibody with which a fluorescent protein obtained by the present invention has been fused emits the fluorescence by itself by means of irradiation of the light having the specific wavelength, for the purpose of detection, the secondary antibody, the tertiary antibody and the like are not required. Therefore, in the case where an antibody obtained by the present invention is utilized for a method for immunological measurement, the simplification of the operational steps and the shortenings of the measurement time periods are accomplished. Moreover, although in the general enzyme immune measurement method, the special reagent (substrate) is required for detection (measurement), in an antibody obtained by the present invention, since the detection is capable of being realized only by irradiation of the light having the specific wavelength, the direct detection can be carried out in a living cell or in a living organism.

Moreover, against different antigens, the multicolor staining is capable of being realized by preparing antibodies with which different fluorescent proteins have been fused and immunostaining using these at the same time.

Furthermore, it is possible that an antigen molecule involved in the specific function in a living cell or in a living organism is measured in real time by combining it with the intra-body technology.

As described above, it can be said that a scFv type antibody with which a fluorescence protein obtained by the present invention has been fused can be utilized for a detecting reagent widely used in the immunostaining field.

## Claims

1. A method for preparing a scFv antibody with which a fluorescent protein has been fused, comprising the following steps:
1) a step of preparing a scFv antibody library composed of phage clones, each one of which expresses a scFv antibody on the surface;
2) a step of selecting a phage clone expressing a scFv antibody capable of binding to the relevant antigen by screening the scFv antibody library with an antigen;
3) a step of acquiring the gene coding for the scFv antibody from the phage clone which has been selected in the step 2);
4) a step of incorporating the relevant gene into an expression vector capable of expressing a fusion protein of the expression product of the relevant gene and a fluorescent protein by incorporating the gene acquired in step 3); and
5) a step of transforming a host using the recombinant vector obtained in step 4) and expressing the fusion protein.

2. The method for preparing a scFv antibody as claimed in Claim 1, wherein the scFv antibody library contains at least one portion of light chain variable region selected so as to reconstruct a functional conformation with a heavy variable region.

3. The method for preparing a scFv antibody as claimed in Claim 1 or 2, wherein the scFv antibody on the surface of each phage clone in the scFv antibody library has a VH region, a VL region, a linker and a CL region.

4. The method for preparing a scFv antibody as claimed in any one of Claim 1 to 3, wherein the fluorescent protein is one or two or more of fluorescent protein(s) selected from the group consisting of GFP, RFP, BFP, YFP, CFP and mutants thereof.

5. A scFv antibody with which a fluorescent protein has been fused, prepared by the method claimed in any one of Claim 1 to 4.

6. A scFv antibody with which a fluorescent protein has been fused, wherein the scFv antibody has a Fv region which consists of a VH region, VL region, and a linker, a CL region, and a fluorescent protein connected to the Fv region via the CL region.

7. The scFv antibody as claimed in Claim 6, wherein the fluorescent protein is one or two or more of fluorescent protein(s) selected from the group consisting of GFP, RFP, BFP, YFP, CFP and mutants thereof.

8. An immunological measurement method using the scFv antibody claimed in any one of Claim5 to 7.

9. A recombinant vector having a base sequence coding for a scFv antibody gene and the base sequence coding for a fluorescent protein, which are acquired by the steps of 1) to 4) claimed in Claim 1.

10. The recombinant vector as claimed in Claim 9, wherein the scFv antibody gene consists of a VH gene, a linker sequence, a VL gene and a CL gene.

11. The recombinant vector as claimed in Claim 9 or 10, wherein the fluorescent protein is one or two or more of fluorescent protein(s) selected from the group consisting of GFP, RFP, BFP, YFP, CFP and mutants thereof.

12. A recombinant vector having the following structure:

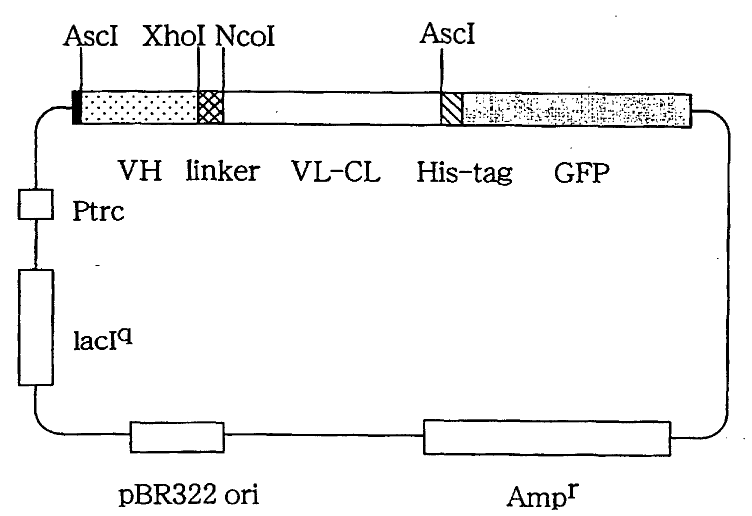

13. An expression vector having a start codon, a site to which a scFv antibody gene is introduced, and base sequence coding for a fluorescent protein in order starting from 5'end side.

14. The expression vector as claimed in Claim 13, wherein the scFv antibody gene consists of a VH gene, a linker sequence, a VL gene and a CL gene.

15. An expression vector as claimed in Claim 13 or 14, wherein the foregoing expression vector has a base sequence coding for His-tag, myc-tag or HA-tag between the site to which the foregoing scFv antibody gene is introduced and the base sequence coding for the foregoing fluorescent protein.

16. The expression vector as claimed in any one of Claime13 to 15, wherein the foregoing fluorescent protein is one or two or more of fluorescent protein(s) selected from the group consisting of GFP, RFP, BFP, YFP, CFP and mutants thereof.

17. A kit for preparing a scFv antibody with which a fluorescent protein has been fused, comprising:
a scFv antibody library composed of phage clones, each one of which expresses a scFv antibody on the surface; and
an expression vector capable of expressing a fusion protein of the expression product of the relevant gene and a fluorescent protein by incorporating the scFv antibody gene.

18. The kit as claimed in Claim 17, wherein the scFv antibody gene consists of aVH region, VL region, a linker sequence, a CL region.

19. A kit for preparing a scFv antibody with which a fluorescent protein is fused, containing a scFv antibody gene library composed of phage clones having a scFv antibody gene or phagemid clones having a scFv antibody gene, and a vector capable of expressing a fusion protein of the expression product of the relevant gene and a fluorescent protein by incorporating a scFv antibody gene.

20. The kit as claimed in Claim 19, wherein the scFv antibody gene consists of a VH gene, a VL gene, a linker sequence and a CL gene.

21. The kit as claimed in any one of Claim 17 to 20, wherein the vector is an expression vector claimed in any one of Claim 13 to 16.


SEQUENCE LISTING

<110> MEDICAL & BIOLOGICAL LABORATORIES CO., LTD.
      Morino, Kazuhiko
      Akahori, Yasushi
      Iba, Yoshitaka
      Shinohara, Midori
      Ukai, Yoshinori
      Kurosawa, Yoshikazu

<120> Method for producing florescent scFv antibody

<130> P020201

<140>
<141>

<150> JP P2000-178880
<151> 2000-06-14

<160> 66

<170> PatentIn Ver. 2.1

<210> 1
<211> 120
<212> PRT
<213> Homo sapiens

<400> 1
Glu Val Gln Leu Val Glu Ser Gly Gly Gly Leu Val Gln Pro Gly Arg
  1               5                  10                  15

Ser Leu Arg Leu Ser Cys Ala Ala Ser Gly Phe Thr Phe Asp Asp Tyr
             20                  25                  30

Ala Met His Trp Val Arg Gln Ala Pro Gly Lys Gly Leu Glu Trp Val
         35                  40                  45

Ser Gly Ile Ser Trp Asn Ser Gly Ser Ile Gly Tyr Ala Asp Ser Val
     50                  55                  60

Lys Gly Arg Phe Thr Ile Ser Arg Asp Asn Ala Lys Asn Ser Leu Tyr
 65                  70                  75                  80

Leu Gln Met Asn Ser Leu Arg Ala Glu Asp Thr Ala Leu Tyr Tyr Cys
                85                  90                  95

Ala Lys Gly Pro Ser Gly Ser Phe Asp Ala Phe Asp Ile Trp Gly Gln
            100                 105                 110

Gly Thr Thr Val Thr Val Ser Ser
        115                 120

<210> 2
<211> 17
<212> DNA
<213> Artificial Sequence

<220>
<223> Description of Artificial Sequence:Artificially
Synthesized Primer Sequence

<400> 2
caggaaacag ctatgac 17

<210> 3
<211> 22
<212> DNA
<213> Artificial Sequence

<220>
<223> Description of Artificial Sequence:Artificially
Synthesized Primer Sequence

<400> 3
cggctccaag tcgacgtcgt ca 22

<210> 4
<211> 83
<212> DNA
<213> Artificial Sequence

<220>
<223> Description of Artificial Sequence:Artificially
Synthesized Primer Sequence

<400> 4
cagctgcagc agtctggggc agagcttgtg aagccagggg cctcagtcaa gttgtcctgc 60
acagcttctg gcttcaacat taa 83

<210> 5
<211> 81
<212> DNA
<213> Artificial Sequence

<220>
<223> Description of Artificial Sequence:Artificially
      Synthesized Primer Sequence

<400> 5
agaccgaagt tgtaatttct gtggatatac gtgacccact tcgtctccgg acttttccca 60
gatctcacct aaccttccta a 81

<210> 6
<211> 84
<212> DNA
<213> Artificial Sequence

<220>
<223> Description of Artificial Sequence:Artificially
      Synthesized Primer Sequence

<400> 6
aagggtctag agtggattgg aaggattgat cctgcgagtg gtaatactaa atatgacccg 60
aaggacaagg ccactataac agca 84

<210> 7
<211> 66
<212> DNA
<213> Artificial Sequence

<220>
<223> Description of Artificial Sequence:Artificially
      Synthesized Primer Sequence

<400> 7
ttcctgttcc ggtgatattg tcgtctgtgt aggaggttgt gtcggatgga tgtcgactta 60
agggac 66

<210> 8
<211> 51
<212> DNA
<213> Artificial Sequence

<220>
<223> Description of Artificial Sequence:Artificially
      Synthesized Primer Sequence

<400> 8
cagctgaatt ccctgacatc tgaggacact gccgtctatt actgtgctgg t          51


<210> 9
<211> 73
<212> DNA
<213> Artificial Sequence

<220>
<223> Description of Artificial Sequence:Artificially
      Synthesized Primer Sequence

<400> 9
cagataatga cacgaccaat actaatgccg ttgaaactga tgaccccggt tccgtggtgc 60
cagtggcaca agg                                                    73


<210> 10
<211> 54
<212> DNA
<213> Artificial Sequence

<220>
<223> Description of Artificial Sequence:Artificially
      Synthesized Primer Sequence

<400> 10
ggttctctaa cagtagtggt agtagtggta attattctcg atagggccct cgaa       54


<210> 11
<211> 69
<212> DNA

<213> Artificial Sequence

<220>
<223> Description of Artificial Sequence:Artificially
      Synthesized Primer Sequence

<400> 11
gacatcgagc tcacccagtc tccagcctcc ctttctgcgt ctgtgggaga aactgtcacc 60
atcacatgt                                                          69


<210> 12
<211> 63
<212> DNA
<213> Artificial Sequence

<220>
<223> Description of Artificial Sequence:Artificially
      Synthesized Primer Sequence

<400> 12
tgacagtggt agtgtacagc tcgttcaccc ttataagtgt taataaatcg taccatggtc 60
gtc                                                                63


<210> 13
<211> 48
<212> DNA
<213> Artificial Sequence

<220>
<223> Description of Artificial Sequence:Artificially
      Synthesized Primer Sequence

<400> 13
gcatggtacc agcagaaacc agggaaatct cctcagctcc tggtctat               48


<210> 14
<211> 81
<212> DNA
<213> Artificial Sequence

<220>
<223> Description of Artificial Sequence:Artificially

Synthesized Primer Sequence

<400> 14
ggagtcgagg accagatatt acgtttttgg aatcgtctac cacacggtag ttccaagtca 60
ccgtcaccta ggccttgtgt t 81

<210> 15
<211> 45
<212> DNA
<213> Artificial Sequence

<220>
<223> Description of Artificial Sequence:Artificially
Synthesized Primer Sequence

<400> 15
tcatgaggca cctgcaagcc acctccgtgg ttcgagctct agttt 45

<210> 16
<211> 45
<212> DNA
<213> Artificial Sequence

<220>
<223> Description of Artificial Sequence:Artificially
Synthesized Primer Sequence

<400> 16
agtactccgt ggacgttcgg tggaggcacc aagctcgaga tcaaa 45

<210> 17
<211> 10
<212> DNA
<213> Artificial Sequence

<220>
<223> Description of Artificial Sequence:Artificially
Synthesized Primer Sequence

<400> 17
atcgacagct 10

<210> 18
<211> 30
<212> DNA
<213> Artificial Sequence

<220>
<223> Description of Artificial Sequence:Artificially
      Synthesized Primer Sequence

<400> 18
aagccacctc catggttcga gctctagttt                                        30


<210> 19
<211> 44
<212> DNA
<213> Artificial Sequence

<220>
<223> Description of Artificial Sequence:Artificially
      Synthesized Primer Sequence

<400> 19
tcgaagttgt ccttactcac aagccgcgcg gtcagctgag gtaa                        44


<210> 20
<211> 55
<212> DNA
<213> Artificial Sequence

<220>
<223> Description of Artificial Sequence:Artificially
      Synthesized Primer Sequence

<400> 20
accctggtca ccgtctcctc agcctccacc aagggcccat cggtcttccc cctgg            55


<210> 21
<211> 37
<212> DNA
<213> Artificial Sequence

<220>
<223> Description of Artificial Sequence:Artificially
      Synthesized Primer Sequence

<400> 21
gggagtcgtc gcagcactgg cacgggaggt cgtcgaa                                37


<210> 22
<211> 36
<212> DNA
<213> Artificial Sequence

<220>
<223> Description of Artificial Sequence:Artificially
      Synthesized Primer Sequence

<400> 22
ggactctact ccctcagcag cgtcgtgacc gtgccc                                 36


<210> 23
<211> 63
<212> DNA
<213> Artificial Sequence

<220>
<223> Description of Artificial Sequence:Artificially
      Synthesized Primer Sequence

<400> 23
gggtcgttgt ggttccacct gttctttcaa ctcgggttta gaacagtagt ggtagtagtg 60
gta                                                                     63


<210> 24
<211> 56
<212> DNA
<213> Artificial Sequence

<220>
<223> Description of Artificial Sequence:Artificially
      Synthesized Primer Sequence

<400> 24

gggtttagaa cagtagtggt agtagtggta attattctcg atagggccct cgaacg     56

<210> 25
<211> 33
<212> DNA
<213> Artificial Sequence

<220>
<223> Description of Artificial Sequence:Artificially
      Synthesized Primer Sequence

<400> 25
ggcaccacgg tcaccgtctc gagcgcctcc acc                              33

<210> 26
<211> 91
<212> DNA
<213> Artificial Sequence

<220>
<223> Description of Artificial Sequence:Artificially
      Synthesized Primer Sequence

<400> 26
caccacggtc accgtctcct caggcggtgg cggatcaggt ggcggtggaa gtggcggtgg 60
tgggtctact agtgacatcg agctcaccca g                                91

<210> 27
<211> 91
<212> DNA
<213> Artificial Sequence

<220>
<223> Description of Artificial Sequence:Artificially
      Synthesized Primer Sequence

<400> 27
gtggtgccag tggcagagga gtccgccacc gcctagtcca ccgccacctt caccgccacc 60
acccagatga tcactgtagc tcgagtgggt c                                91

<210> 28

<211> 17
<212> DNA
<213> Artificial Sequence

<220>
<223> Description of Artificial Sequence:Artificially
      Synthesized Primer Sequence

<400> 28
caggaaacag ctatgac                                                17


<210> 29
<211> 42
<212> DNA
<213> Artificial Sequence

<220>
<223> Description of Artificial Sequence:Artificially
      Synthesized Primer Sequence

<400> 29
gacgccgggt cggccggtac cggctccaag tcgacgtcgt ca                    42


<210> 30
<211> 56
<212> DNA
<213> Artificial Sequence

<220>
<223> Description of Artificial Sequence:Artificially
      Synthesized Primer Sequence

<400> 30
gtcctcgcaa ctgcggccca gccggccatg gccgacatcc agatgaccca gtctcc      56


<210> 31
<211> 56
<212> DNA
<213> Artificial Sequence

<220>
<223> Description of Artificial Sequence:Artificially

Synthesized Primer Sequence

<400> 31
gtcctcgcaa ctgcggccca gccggccatg gccgatgttg tgatgactca gtctcc     56


<210> 32
<211> 56
<212> DNA
<213> Artificial Sequence

<220>
<223> Description of Artificial Sequence:Artificially
      Synthesized Primer Sequence

<400> 32
gtcctcgcaa ctgcggccca gccggccatg gccgaaattg tgttgacgca gtctcc     56


<210> 33
<211> 56
<212> DNA
<213> Artificial Sequence

<220>
<223> Description of Artificial Sequence:Artificially
      Synthesized Primer Sequence

<400> 33
gtcctcgcaa ctgcggccca gccggccatg gccgacatcg tgatgaccca gtctcc     56


<210> 34
<211> 56
<212> DNA
<213> Artificial Sequence

<220>
<223> Description of Artificial Sequence:Artificially
      Synthesized Primer Sequence

<400> 34
gtcctcgcaa ctgcggccca gccggccatg gccgaaacga cactcacgca gtctcc     56

<210> 35
<211> 56
<212> DNA
<213> Artificial Sequence

<220>
<223> Description of Artificial Sequence:Artificially
      Synthesized Primer Sequence

<400> 35
gtcctcgcaa ctgcggccca gccggccatg gccgaaattg tgctgactca gtctcc     56


<210> 36
<211> 56
<212> DNA
<213> Artificial Sequence

<220>
<223> Description of Artificial Sequence:Artificially
      Synthesized Primer Sequence

<400> 36
gtcctcgcaa ctgcggccca gccggccatg gcccagtctg tgttgacgca gccgcc     56


<210> 37
<211> 56
<212> DNA
<213> Artificial Sequence

<220>
<223> Description of Artificial Sequence:Artificially
      Synthesized Primer Sequence

<400> 37
gtcctcgcaa ctgcggccca gccggccatg gcccagtctg ccctgactca gcctgc     56


<210> 38
<211> 56
<212> DNA
<213> Artificial Sequence

<220>

<223> Description of Artificial Sequence:Artificially
      Synthesized Primer Sequence

<400> 38
gtcctcgcaa ctgcggccca gccggccatg gcctcctatg tgctgactca gccacc      56


<210> 39
<211> 56
<212> DNA
<213> Artificial Sequence

<220>
<223> Description of Artificial Sequence:Artificially
      Synthesized Primer Sequence

<400> 39
gtcctcgcaa ctgcggccca gccggccatg gcctcttctg agctgactca ggaccc      56


<210> 40
<211> 56
<212> DNA
<213> Artificial Sequence

<220>
<223> Description of Artificial Sequence:Artificially
      Synthesized Primer Sequence

<400> 40
gtcctcgcaa ctgcggccca gccggccatg gcccacgtta tactgactca accgcc      56


<210> 41
<211> 56
<212> DNA
<213> Artificial Sequence

<220>
<223> Description of Artificial Sequence:Artificially
      Synthesized Primer Sequence

<400> 41
gtcctcgcaa ctgcggccca gccggccatg gcccaggctg tgctcactca gccgcc      56

<210> 42
<211> 56
<212> DNA
<213> Artificial Sequence

<220>
<223> Description of Artificial Sequence:Artificially
      Synthesized Primer Sequence

<400> 42
gtcctcgcaa ctgcggccca gccggccatg gccaatttta tgctgactca gcccca     56

<210> 43
<211> 44
<212> DNA
<213> Artificial Sequence

<220>
<223> Description of Artificial Sequence:Artificially
      Synthesized Primer Sequence

<400> 43
tcgactggcg cgccgaacac tctcccctgt tgaagctctt tgtg     44

<210> 44
<211> 43
<212> DNA
<213> Artificial Sequence

<220>
<223> Description of Artificial Sequence:Artificially
      Synthesized Primer Sequence

<400> 44
tcgactggcg cgccgaacat tctgtagggg ccactgtctt ctc     43

<210> 45
<211> 20
<212> DNA
<213> Artificial Sequence

<220>
<223> Description of Artificial Sequence:Artificially
      Synthesized Primer Sequence

<400> 45
attaataaga gctatcccgg                                                    20


<210> 46
<211> 20
<212> DNA
<213> Artificial Sequence

<220>
<223> Description of Artificial Sequence:Artificially
      Synthesized Primer Sequence

<400> 46
atggagtcgg gaaggaagtc                                                    20


<210> 47
<211> 56
<212> DNA
<213> Artificial Sequence

<220>
<223> Description of Artificial Sequence:Artificially
      Synthesized Primer Sequence

<400> 47
gtcctcgcaa ctgcggccca gccggccatg gcccaggtgc agctggtgca gtctgg      56


<210> 48
<211> 56
<212> DNA
<213> Artificial Sequence

<220>
<223> Description of Artificial Sequence:Artificially
      Synthesized Primer Sequence

<400> 48
gtcctcgcaa ctgcggccca gccggccatg gcccaggtca acttaaggga gtctgg      56

<210> 49
<211> 56
<212> DNA
<213> Artificial Sequence

<220>
<223> Description of Artificial Sequence:Artificially
      Synthesized Primer Sequence

<400> 49
gtcctcgcaa ctgcggccca gccggccatg gccgaggtgc agctggtgga gtctgg     56


<210> 50
<211> 56
<212> DNA
<213> Artificial Sequence

<220>
<223> Description of Artificial Sequence:Artificially
      Synthesized Primer Sequence

<400> 50
gtcctcgcaa ctgcggccca gccggccatg gcccaggtgc agctgcagga gtcggg     56


<210> 51
<211> 56
<212> DNA
<213> Artificial Sequence

<220>
<223> Description of Artificial Sequence:Artificially
      Synthesized Primer Sequence

<400> 51
gtcctcgcaa ctgcggccca gccggccatg gcccaggtgc agctgttgca gtctgc     56


<210> 52
<211> 56
<212> DNA
<213> Artificial Sequence

<220>
<223> Description of Artificial Sequence:Artificially
      Synthesized Primer Sequence

<400> 52
gtcctcgcaa ctgcggccca gccggccatg gcccaggtac agctgcagca gtcagg     56


<210> 53
<211> 59
<212> DNA
<213> Artificial Sequence

<220>
<223> Description of Artificial Sequence:Artificially
      Synthesized Primer Sequence

<400> 53
gtcctcgcaa ctgcggccca gccggccatg gcccagrtca ccttgaagga gtctggtcc  59


<210> 54
<211> 56
<212> DNA
<213> Artificial Sequence

<220>
<223> Description of Artificial Sequence:Artificially
      Synthesized Primer Sequence

<400> 54
gtcctcgcaa ctgcggccca gccggccatg gcccaggtgc agctacagca gtgggg     56


<210> 55
<211> 56
<212> DNA
<213> Artificial Sequence

<220>
<223> Description of Artificial Sequence:Artificially
      Synthesized Primer Sequence

<400> 55

gtcctcgcaa ctgcggccca gccggccatg gccgaggtgc agctggtgca gtctgg     56

<210> 56
<211> 64
<212> DNA
<213> Artificial Sequence

<220>
<223> Description of Artificial Sequence:Artificially
      Synthesized Primer Sequence

<400> 56
gtcctcgcaa ctgcggccca gccggccatg gcccaggtgc agctggtgca atctgggtct 60
gagt                                                              64

<210> 57
<211> 32
<212> DNA
<213> Artificial Sequence

<220>
<223> Description of Artificial Sequence:Artificially
      Synthesized Primer Sequence

<400> 57
ggtggaggca ctcgagacgg tgaccagggt gc                               32

<210> 58
<211> 32
<212> DNA
<213> Artificial Sequence

<220>
<223> Description of Artificial Sequence:Artificially
      Synthesized Primer Sequence

<400> 58
ggtggaggca ctcgagacgg tgaccattgt cc                               32

<210> 59
<211> 32

<212> DNA
<213> Artificial Sequence

<220>
<223> Description of Artificial Sequence:Artificially
      Synthesized Primer Sequence

<400> 59
ggtggaggca ctcgagacgg tgaccagggt tc                                32


<210> 60
<211> 32
<212> DNA
<213> Artificial Sequence

<220>
<223> Description of Artificial Sequence:Artificially
      Synthesized Primer Sequence

<400> 60
ggtggaggca ctcgagacgg tgaccgtggt cc                                32


<210> 61
<211> 77
<212> DNA
<213> Artificial Sequence

<220>
<223> Description of Artificial Sequence:Artificially
      Synthesized Primer Sequence

<400> 61
gtcaccgtct cgagaggcgg tggcggatca ggtggcggtg gaagtggcgg tggtgggtcc 60
atggccgaca tcgagct                                                 77


<210> 62
<211> 68
<212> DNA
<213> Artificial Sequence

<220>
<223> Description of Artificial Sequence:Artificially

Synthesized Primer Sequence

<400> 62
cgatgtcggc catggaccca ccaccgccac ttccaccgcc acctgatccg ccaccgcctc 60
tcgagacg 68


<210> 63
<211> 27
<212> DNA
<213> Artificial Sequence

<220>
<223> Description of Artificial Sequence:Artificially
Synthesized Primer Sequence

<400> 63
   taggcgcgcc aggggggttc tcatcat 27


<210> 64
<211> 27
<212> DNA
<213> Artificial Sequence

<220>
<223> Description of Artificial Sequence:Artificially
Synthesized Primer Sequence

<400> 64
aaggcgcgcc atggtttatt cctcctt 27


<210> 65
<211> 31
<212> DNA
<213> Artificial Sequence

<220>
<223> Description of Artificial Sequence:Artificially
Synthesized Primer Sequence

<400> 65
aaggcgcgcc aggtgcagct ggtggagtct g 31

<210> 66
<211> 15
<212> DNA
<213> Artificial Sequence

<220>
<223> Description of Artificial Sequence:Artificially
      Synthesized Primer Sequence

<400> 66
gtcgactggc gcgcc                                                    15